# EUROPEAN PATENT APPLICATION

(11) **EP 3 950 934 A1**
(43) Date of publication of application: **09.02.2022**
(21) Application number: 20778085.9
(22) Date of filing: 25.03.2020
(51) Int. Cl.: C12N 5/077

(54) **METHOD FOR CULTURING CELL POPULATION AND USE THEREOF**

(30) Priority: 25.03.2019 WO PCT/JP2019/012571
(71) Applicant: TOKAI UNIVERSITY EDUCATIONAL SYSTEM, Tokyo 151-0063 (JP); NIPPON ZOKI PHARMACEUTICAL CO., LTD., Chuo-ku Osaka-shi Osaka 541-0046 (JP)
(72) Inventor: SAKAI Daisuke, Isehara-shi, Kanagawa 259-1193 (JP); NAKAMURA Yoshihiko, Isehara-shi, Kanagawa 259-1193 (JP); MATSUSHITA Erika, Isehara-shi, Kanagawa 259-1193 (JP)
(74) Representative: Hoffmann Eitle
(86) International application number: PCT/JP2020/013307
(87) International publication number: WO 2020/196615

(57) **Abstract**

The present invention addresses the problem of providing a means for efficiently preparing a cell population rich in cells having a given phenotype depending on their use (e.g., type II collagen-positive nucleus pulposus cells) from a cell population containing Tie2-positive stem/progenitor cells (e.g., nucleus pulposus stem/progenitor cells). The present invention provides culture methods wherein a cell population containing Tie2-positive stem/progenitor cells is cultured (1) while present in a non-digested tissue, (2) in a culture medium containing at least one kind of Tie2 expression enhancer other than growth factors, (3) using cultureware with a culture surface having undergone cell attachment-increasing treatment, or (4) while suppressing formation of spheroid colonies in a culture medium containing an extracellular matrix-degrading agent.

## Description

### [Technical Field]

The present invention relates to, among methods of culturing a cell population, for instance, a method of culturing a cell population containing stem cells and/or progenitor cells positive for expression of a cell surface marker Tie2 (tyrosine kinase with Ig and EGF homology domain-2) (herein referred to as "Tie2-positive stem/progenitor cells"). More specifically, the present invention relates to, for instance, a method of culturing a cell population containing Tie2-positive stem/progenitor cells capable of being used in a step of amplifying Tie2-positive stem/progenitor cells (e.g., nucleus pulposus stem/progenitor cells) in a cell population and/or a step of inducing differentiation from Tie2-positive stem/progenitor cells into cells having a given phenotype (e.g., type II collagen-expressing nucleus pulposus cells).

### [Background Art]

In this country, low back pain is ranked second in the prevalence, is a common disease so that 2/3 of the adult population experiences at least once in their lifetime, and is a cause of work-related disorders and/or social problems in the medical economy. Disc disorder, which is said to account for 20% of low back pain, causes serious problems that can induce, for instance, disc herniation, spondylosis deformans, spinal stenosis, or spondylolisthesis. These are due to irreversible changes in the disc tissue, and are pathological conditions called intervertebral disc degeneration. The intervertebral disc is a donut-shaped cartilaginous organ comprising a nucleus pulposus (NP) in the center, an annulus fibrosus (AF), namely multi-layered fibrous cartilage surrounding the nucleus pulposus, and a cartilaginous endplate (EP) vertically connecting adjacent vertebrae. A gelatinous nucleus pulposus is an avascular organ rich in an extracellular matrix (ECM) composed of large proteoglycan and collagen secreted from nucleus pulposus cells derived from the notochord contained in the nucleus pulposus. In some vertebrates including humans, notochord-derived nucleus pulposus cells reportedly disappear early in life. After the disappearance, the nucleus pulposus is formed by chondroid cells of unknown origin that are morphologically similar to chondrocytes. Such a phenotypic cell change affects the ECM composition, causes disc aging and/or degeneration such as a decrease in moisture content or fibrosis, and seems to be finally significantly involved in low back pain and/or lumbar degenerative diseases. Note that many animal species such as a mouse, a rat, a rabbit, and a pig have, through their lifetime, the notochord-derived nucleus pulposus cells, and intervertebral disc degeneration is hardly seen. This may be because the mechanism of regulating the notochord-derived nucleus pulposus cells and other nucleus pulposus cells is different from that of human.

As an example of a protocol for preventing or treating intervertebral disc degeneration, R&D of an allogeneic disc cell preparation is in progress, including a cell preparation containing, for instance, allogeneic nucleus pulposus cells and ECM for administration to an intervertebral disc tissue. Production of such a cell preparation requires a certain amount of allogeneic nucleus pulposus cells. For example, a disc nucleus pulposus tissue excised from a patient with disc herniation by surgery can be utilized as a source of nucleus pulposus cells for use in such a cell preparation. However, the amount of nucleus pulposus tissue that can be collected in such a manner, that is, the number of nucleus pulposus cells contained therein is limited. Meanwhile, in view of the risk of viral infection, for instance, it is desirable to avoid use of a mixture of nucleus pulposus cells derived from multiple patients (donors) with disc herniation in order to secure the number of nucleus pulposus cells. Thus, it is critical to establish a technology for preparing a cell population containing a sufficient number of nucleus pulposus cells for treatment by culturing rare stem cells or progenitor cells that are contained in a small a volume of disc tissue (e.g., the nucleus pulposus, AF) derived from a single donor and can be differentiated into mature nucleus pulposus cells.

Patent Document 1 discloses production of a "discosphere" comprising stem cells and progenitor cells contained in a cell population by culturing nucleus pulposus cells (cell population derived from the intervertebral disc nucleus pulposus) "under cell attachment-interfering conditions" (preferably by culturing in a serum-free culture medium). That is, Patent Document 1 describes a "method of producing a disc stem cell population", including the steps of: growing nucleus pulposus cells in a culture medium "under cell attachment-interfering conditions"; (b) concentrating disc stem cells, disc progenitor cells, or a combination thereof; and (c) producing a discosphere including nucleus pulposus cells, thereby producing a disc stem cell population (e.g., claim 3). Note that the "discosphere" is described as comprising floating nucleus pulposus stem cells and nucleus pulposus cells arranged in a circular-spherical structure, such as an *in vitro* free floating circular-spherical structure comprising disc stem cells, disc progenitor cells, or a combination thereof or a ball of cells in which a single disc stem cell gives rise to clones of itself and to progenitor cells; or is described such that the nucleus pulposus cells comprising a discosphere are attached to each other (paragraphs [0024] and [0039]). Patent Document 1 further discloses "an isolated disc stem cell population" that is cultured "under cell attachment-interfering conditions" and is obtained by enriching disc stem cells, disc progenitor cells, or a combination thereof (e.g., claim 1); "an isolated discosphere that comprises disc stem cells, disc progenitor cells, or a mixture thereof enriched from nucleus pulposus cells and is an *"in vitro* floating spherical structure (e.g., claim 10); "an artificial disc replacement device" comprising a disc scaffold and a discosphere comprising nucleus pulposus cells obtained by enriching disc stem cells, disc progenitor cells, or a combination thereof (e.g., claim 11); "a method of producing an artificial disc replacement device, comprising the step of growing, in a disc scaffold, discospheres comprising disc stem cells, disc progenitor cells, or a mixture thereof enriched from nucleus pulposus cells (e.g., claim 12); a method of "producing an enriched cell population", comprising the steps of: culturing nucleus pulposus cells plated at a given low density "under cell attachment-interfering conditions"; and selecting an *in vitro* "floating spherical structure" comprising disc stem cells, disc progenitor cells, or a mixture thereof, thereby producing an enriched cell population (e.g., claim 17); "a method of amplifying a population containing enriched disc stem cells, disc progenitor cells, or a combination thereof', comprising the steps of: dissociating disc stem cells, disc progenitor cells, or a combination thereof into one or more dissociated disc cells; and culturing the one or more dissociated disc cells in a medium that contains predetermined additives (e.g., FGF2, EGF) and "interferes with cell attachment" (e.g., claim 18); and so on. Note that "disc" in Patent Document 1 is considered to be a direct translation of the original word "disc" and means "intervertebral disc".

The following can be said about the matters related to cultureware or culture media for culturing a heterogeneous cell population (nucleus pulposus-derived cell population) including, for instance, disc stem cells, disc progenitor cells, and disc cells derived from the disc nucleus pulposus tissue as disclosed in Patent Document 1.

Patent Document 1 discloses an embodiment in which, as culture "under cell attachment-interfering conditions", culture by plating cells at a low density in a serum-free medium containing a substance (specifically, methylcellulose) interfering with cell attachment or culture using a ultra-low-attachment plate is conducted to produce a discosphere, namely a floating spherical structure, from a nucleus pulposus-derived cell population (e.g., stem cells included therein) (see paragraph [0156] and the following, and Example 1: paragraphs [0170] to [0181], corresponding to the inventions of claims 3, 17, and others). However, Patent Document 1 neither describes nor suggests that a nucleus pulposus-derived cell population (e.g., stem cells contained therein) is cultured in a medium containing a substance (e.g., collagenase) that degrades the extracellular matrix or on a cell-adherent culture surface, and the disc stem cells are amplified or differentiated without formation of discospheres (floating spherical structures).

In addition, Patent Document 1 discloses, as a method of amplifying a cell population containing, for instance, enriched disc stem cells, an embodiment in which the discospheres (floating spherical structures) are first dissociated into one or more disc stem cells by incubation in a medium supplemented with collagenase, and then the dissociated cells are re-plated in a methylcellulose-containing medium (see paragraph [0157] and Example 2: paragraphs [0182] to [0184], corresponding to the inventions of claims 18 and others). However, the culture in the medium supplemented with collagenase in this embodiment is just temporary treatment for dissociating the discospheres once formed into, for instance, individual disc stem cells. The treatment is not for inducing differentiation of disc stem cells and others, and the dissociated disc stem cells and others are re-cultured "under cell attachment-interfering conditions" (e.g., in a methylcellulose-containing medium). Patent Document 1 neither describes nor suggests that culturing is started in a medium supplemented with collagenase while using disc stem cells or others in a state in which no discosphere is formed (before formation), and the disc stem cells or others are amplified (grown) or differentiated, or that culturing is made to continue in a medium supplemented with collagenase even after the disc stem cells or others are dissociated from each other, and the disc stem cells and others are amplified (grown) and differentiated while keeping a state in which no discosphere, i.e., no floating spherical structure, is formed.

Note that Patent Document 1 describes growing disc stem cells in a serum-free medium containing a "compound that inhibits maturation of cells" (e.g., FGF) or a "compound that maintains immaturity of cells" (e.g., each TGF-β superfamily member, BMP, IL-6, LIF) (paragraphs [0035] to [0037]). However, Patent Document 1 neither describes nor suggests culturing disc stem cells in a medium containing a substance that promotes activation of Tie2.

Further, Patent Document 1 describes, as a method of preparing a nucleus pulposus tissue to obtain a nucleus pulposus-derived cell population, only a general technique of fragmenting a nucleus pulposus tissue by fragmenting the nucleus pulposus (surgically obtained human disc material or biopsy specimen) and treating the nucleus pulposus tissue with, for instance, collagenase II or *Clostridium* collagenase to dissociate individual cells (to prepare a single cell suspension) (e.g., paragraphs [0026], [0029], and [0030], Example 1: paragraphs [0171] to [0174]).

Meanwhile, Patent Document 2 and Non-Patent Document 1 disclose that among cells contained in an intervertebral disc tissue (nucleus pulposus), cells positive for Tie2 and/or GD2 as a cell surface marker are cells that can be called stem cells or progenitor cells of nucleus pulposus cells; in particular, cells positive for both Tie2 and GD2 (nucleus pulposus stem cells in an active state) form spheroid colonies and have a potential of finally differentiating into mature nucleus pulposus cells through a series of differentiation cascades (in addition, have a potential of differentiating into adipocytes, osteocytes, chondrocytes, and neurons); and further, implantation of nucleus pulposus stem/progenitor cells into an intervertebral disc (nucleus pulposus) makes it possible to produce an extracellular matrix such as type II collagen in the tissue, maintain or reconstruct the intervertebral disc tissue, and prevent or treat intervertebral disc degeneration.

As a more specific embodiment, Patent Document 2 and Non-Patent Document 1 disclose that spheroid colonies were formed (together with adherent colonies) by subjecting a cell population contained in an intervertebral disc tissue (nucleus pulposus) to suspension culture in a methylcellulose medium; such spheroid colonies are derived from the Tie2-positive (and GD2-positive) cells described above; and type II collagen and proteoglycan are expressed in spheroid colonies (some cells thereof) (see, for example, Examples, paragraphs [0067] and [0070], and others of Patent Document 2). However, Patent Document 2 or Non-Patent Document 1 neither describes nor suggests that nucleus pulposus stem/progenitor cells (Tie2- and/or GD2-positive cells) are amplified or differentiated without forming spheroid colonies in a medium containing a substance (e.g., collagenase) that degrades an extracellular matrix or on a cell-adherent culture surface (using a methylcellulose-free medium).

Further, Patent Document 2 and Non-Patent Document 1 also describe, as a method of preparing a cell population derived from a nucleus pulposus tissue of an intervertebral disc, only a general technique in which a tissue is fragmented with, for instance, scissors and then digested with a protease (e.g., TrypLE Express, Collagenase P) (Examples: paragraph [0048]).

Note that Patent Document 2 and Non-Patent Document 1 disclose that in order to maintain Tie2-positive nucleus pulposus cells (disc nucleus pulposus stem/progenitor cells), a signaling mechanism between Tie2 (a receptor) and Ang-1 (Angiopoietin-1, a ligand) is required; and Tie2-positive cells can be amplified by culturing in the presence of Ang-1 (co-culturing with AHESS 5 forcibly expressing Ang-1), and Ang-1 is thus considered to be a niche factor that controls the differentiation hierarchy of nucleus pulposus cells (Examples: paragraphs [0049], [0069], [0075], and others).

By the way, Tie2 is also expressed in vascular endothelial cells, and it is known that when Tie2 is activated, maturation, normalization, or stabilization of blood vessels is brought about, for example, disorganized blood vessel expansion (angiogenesis) observed in, for instance, tumors, rheumatoid arthritis, diabetic retinopathy, hyperlipidemia, or hypertension can be suppressed, and wrinkles can be prevented and improved. Examples of the Tie2 activator having such an action include an extract derived from a plant of the genus *Cinnamomum* (what is called cinnamon powder, Patent Document 3) or an olive fruit extract (Patent Document 4) as well as various extracts derived from animals/plants such as quillaja, yellow wood, ginkgo, oysters, turmeric, chrysanthemum, jujube, Chinese matrimony vine, camomile, butcher bloom, hawthorn, star fruit, *Alpinia speciosa,* lotus, rooibos, *Tamarindus indica L.,* Chinese quince, *Psidium guajava,* long pepper, Siberian ginseng, mango ginger, Panax ginseng, *Elaeagnus umbellata, Salsola komarovii, Kalopanax pictus,* Japanese clethra, *Hemerocallis fulva var. kwanso, Colocasia gigantea, Staphylea pinnata, Clerodendrum trichotomum, Stauntonia hexaphylla, Pellionia minima, Quercus serrata, Quercus acutissima, Lactuca indica,* star apple, psyllium, wild rocambole, *Myrica rubra, Gleditsia officinalis Hemsl., Polygonatum rhizome, Polygonatum odoratum,* trichosanthes seed, or *Morinda officinalis* (Patent Documents 5 to 11). Further, examples of a disclosed component that brings about the Tie2 activation/effect include ursolic acid, colosolic acid, 3-O-galloylprocyanidin B-1, linolenic acid, 13-hydroxy-9Z,11E,15E-octadecatrienoic acid, procyanidin B-2, epicatechin-(4β-6)-epicatechin(4β-8)-epicatechin, procyanidin C-1, astragaloside VIII, soya saponin I, 3'-O-methyl gallocatechin, pipernonaline, syringaresinol, 2-methoxycinnamaldehyde, eleutheroside E, eleutheroside E1, sesamin, eudesmin, sylvatesmin, pinoresinol, yangambin, forsythinol, or coumarin (Patent Documents 6 and 12 to 14).

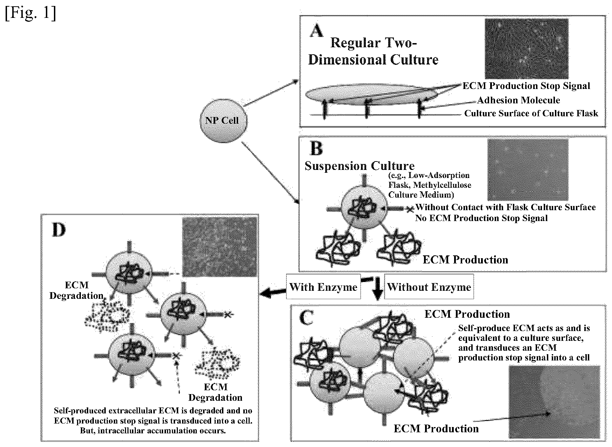

For example, Patent Document 3 provides an experiment (Examples) about the "Tie2 activation agent" such that when "hematopoietic Baf3 cells forcibly expressing Tie2" or "normal human umbilical vein endothelial cells (HUVEC)" were cultured in a medium containing a cinnamon twig hot water extract, the Tie2 protein expressed in these cells was found to be more phosphorylated than that in the control by Western blotting (e.g., paragraphs [0024] to [0027], Figs. 1 to 3).

However, Patent Document 3 to 14 neither describe nor suggest use of the Tie2 activation agent in the culture of a nucleus pulposus-derived cell population (including Tie2-positive stem/progenitor cells) obtained from an intervertebral disc, or what kinds of effects are exerted thereby.

### [Prior Art Documents]

### [Patent Documents]

Patent Document 1: Japanese Patent No. 5509073 (corresponding to WO 2009/009020)
Patent Document 2: Japanese Patent No. 5863639 (corresponding to WO 2011/122601)
Patent Document 3: Japanese Patent Laid-Open No. 2009-263358 (related to WO 2009/123211)
Patent Document 4: WO2016/060249
Patent Document 5: WO2012/073627
Patent Document 6: Japanese Patent Laid-Open No. 2012-236795
Patent Document 7: Japanese National-Phase Publication No. 2009-154237
Patent Document 8: Japanese Patent Laid-Open No. 2011-201811
Patent Document 9: Japanese Patent Laid-Open No. 2011-102275
Patent Document 10: Japanese Patent Laid-Open No. 2011-102274
Patent Document 11: Japanese Patent Laid-Open No. 2011-102273
Patent Document 12: Japanese Patent Laid-Open No. 2014-97977
Patent Document 13: Japanese Patent Laid-Open No. 2013-241356
Patent Document 14: Japanese Patent Laid-Open No. 2011-102272

### [Non-Patent Documents]

Non-Patent Document 1: Sakai D et al., Nat Commun. 2012; 3: 1264

### [Summary of the Invention]

### [Problem to be Solved by the Invention]

As described above, in order to produce an allogenic disc cell preparation for prevention or treatment of, for instance, intervertebral disc degeneration, it is necessary to have some amount of functional nucleus pulposus cells that produce an extracellular matrix such as type II collagen and proteoglycan. For this purpose, a large volume of functional nucleus pulposus cells should be produced by efficiently amplifying and differentiating Tie2-positive cells, which are considered stem cells and/or progenitor cells of nucleus pulposus cells included in an intervertebral disc tissue (e.g., a nucleus pulposus) excised from, for instance, a lesion of a patient with disc herniation. In particular, in order to enhance the therapeutic effect when a cell preparation is administered to a patient with, for instance, intervertebral disc degeneration, it is important not to simply differentiate into nucleus pulposus cells but to differentiate into functional nucleus pulposus cells that produce a large amount of extracellular matrix such as type II collagen as efficiently as possible when the Tie2-positive cells (nucleus pulposus stem/progenitor cells) contained in the intervertebral disc are cultured and differentiated. At the same time, it is also important to efficiently amplify Tie2-positive cells (nucleus pulposus stem/progenitor cells) in the cell population obtained from the tissue in advance before differentiated as described above in order to increase the number of finally produced functional nucleus pulposus cells. That is, there is a need for a practical means for efficiently amplifying and differentiating Tie2-positive cells (nucleus pulposus stem/progenitor cells) from a cell population containing a certain number of Tie2-positive cells (nucleus pulposus stem/progenitor cells), thereby enriching functional nucleus pulposus cells in a finally prepared cell preparation (cell population) to be administered.

In addition, in a typical embodiment of the prior art as described in Patent Documents 1 and 2, spheroid colonies (discospheres, spheroids) are formed by culturing a cell population containing nucleus pulposus stem/progenitor cells included in an intervertebral disc tissue in a methylcellulose-containing medium, and the cell population is then differentiated into nucleus pulposus cells. However, since methylcellulose is a highly viscous substance, it is difficult or requires a great deal of labor to recover, without waste, a cell population (including useful functional nucleus pulposus cells) generated in a methylcellulose-containing medium. This is an obstacle for efficient production and practical use of the cell preparation.

The present invention addresses the problem of providing a means for efficiently preparing a cell population rich in cells having a given phenotype depending on their use (e.g., functional nucleus pulposus cells that produce an extracellular matrix such as type II collagen) from a cell population containing stem cells and/or progenitor cells positive for expression of Tie2 (e.g., a cell population comprising nucleus pulposus stem/progenitor cells derived from an intervertebral disc).

### [Means for Solving the Problems]

The present inventors have conducted research, focusing on the state of a cell population to be cultured and its culture conditions when a cell population containing Tie2-positive cells (nucleus pulposus stem/progenitor cells) included in an intervertebral disc nucleus pulposus tissue is cultured, and as a result, have found a plurality of technical features that can contribute to providing a solution to the above problems. Among culture methods having these technical features, one culture method is very useful in a culturing step at a stage (amplification culture stage) mainly aimed at amplifying nucleus pulposus stem/progenitor cells, and another culture method is very useful in a culturing step at a stage (differentiation culture stage) mainly aimed at inducing differentiation from nucleus pulposus stem/progenitor cells into functional nucleus pulposus cells. Further, it has also been found that these methods may be used in combination sequentially or simultaneously, and in particular, the effects of the invention can be synergistically elicited by adopting a culturing step in which these culture methods are "fused" and implemented simultaneously.

Specifically, an aspect of the invention provides the following first to fourth culture methods that can be combined (preferably fused) as a method of culturing a cell population containing Tie2-positive stem/progenitor cells represented by Tie2-positive cells (nucleus pulposus stem/progenitor cells) included in an intervertebral disc tissue.

### First Culture Method

The "first culture method" for a cell population containing Tie2-positive stem/progenitor cells according to the invention is a method of culturing a cell population containing Tie2-positive stem/progenitor cells while present in a non-digested tissue.

Conventionally, when trying to culture a cell population contained in a nucleus pulposus tissue of an intervertebral disc, it has been common to finely cut the nucleus pulposus tissue of the collected intervertebral disc, perform a tissue-digesting treatment (digestion treatment) with a protein-degrading enzyme (protease) such as collagenase, and then recover the cell population separated from the nucleus pulposus tissue by the treatment to start culture. However, the present inventors have found that in a case where a nucleus pulposus tissue is finely cut, the finely cut nucleus pulposus tissue without digestion treatment is suspended in a culture medium, and the cell population is cultured for a certain period while being kept in the tissue, the ratio of Tie2-positive stem/progenitor cells (nucleus pulposus stem/progenitor cells) in the cell population is increased and the expression of Tie2 in individual cells is also enhanced more than in a case where conventional digestion treatment is performed. Such effects are preferable for the nucleus pulposus stem/progenitor cells because the nucleus pulposus tissue is not digested. That is, growth factors such as Angiopoietin-1 (Ang -1) and VEGF-A are present, and the tissue microenvironment (niche) in which Tie2-positive cells are maintained is not destroyed (without, for instance, Ang-1, Tie2-positive cells eventually undergo apoptosis). A cell population containing the nucleus pulposus stem/progenitor cells having kept in such a niche is used to start culture. This enables the nucleus pulposus stem/progenitor cells, in which the Tie2 activity is maintained (i.e., expression of Tie2 is augmented more than that by a conventional method of isolating cells from a niche), to start growing rapidly. Thus, the positive rate and the level of expression should be increased in the cell population obtained after the culture.

In addition, since the operation of separating and recovering the cell population from the nucleus pulposus tissue is unnecessary, valuable Tie2-positive stem/progenitor cells contained in the nucleus pulposus tissue can be used without waste. For example, the amount of nucleus pulposus tissue contained in a herniated lesion excised from a patient with disc herniation is about 1 to 2 g at most. The number of Tie2-positive stem/progenitor cells contained per g of the nucleus pulposus tissue varies depending on, for instance, the age of the patient, and is, for example, about 50,000. The number of leukocytes contained in 1 cc of umbilical cord blood is on the order of 10⁶; and the number of cancer cells contained per g of cancer tissue is on the order of 10⁸.In view of this comparison, one can understand how valuable the Tie2-positive stem/progenitor cells in the nucleus pulposus tissue are. The first culture method is very advantageous because the number of such Tie2-positive stem/progenitor cells can be prevented from being decreased through the operation of separating and recovering the cell population from the nucleus pulposus tissue, and this is preferable for amplification culture of the Tie2-positive stem/progenitor cells.

### Second Culture Method

The "second culture method" for a cell population containing Tie2-positive stem/progenitor cells according to the invention is a method of culturing a cell population containing Tie2-positive stem/progenitor cells in a culture medium containing at least one kind of Tie2 expression enhancer other than growth factors.

It has been known that in Tie2-positive stem/progenitor cells, Angiopoietin-1 (Ang-1), an intrinsic ligand for Tie2 (receptor tyrosine kinase), is bound to promote Tie2 activation (phosphorylation). A procedure for culturing Tie2-positive stem/progenitor cells in an Ang-1-containing culture medium (i.e., a procedure for enhancing expression of Tie2) is a known conventional technology (e.g., in the prior art literatures). Similarly, FGF2 (bFGF) is also known as a growth factor having an action of enhancing the expression of Tie2, and a method of culturing Tie2-positive stem/progenitor cells in a FGF2-containing culture medium is also known.

However, the present inventors have utilized a Tie2 expression enhancer, the kind of which is different from growth factors such as Ang-1 and FGF2, for example, a Tie2 expression enhancer that is a plant-derived extract such as cinnamon powder extract, for culturing a cell population containing Tie2-positive stem/progenitor cells derived from a nucleus pulposus tissue, which has not been reported so far. In particular, the Tie2 expression enhancer that is a plant-derived extract has been used in combination with a growth factor(s) such as FGF2. In this case, it has been found that there is a remarkable effect such as an increase in the ratio of Tie2-positive stem/progenitor cells (nucleus pulposus stem/progenitor cells) in the cell population obtained by the culture.

The present inventors have further found that a cell population having a high cell number or ratio of Tie2-positive stem/progenitor cells (nucleus pulposus stem/progenitor cells) can be obtained synergistically from a cell population containing Tie2-positive stem/progenitor cells (nucleus pulposus stem/progenitor cells) in a nucleus pulposus tissue by combining (particularly fusing) the above-described "first culture method" and "second culture method".

### Third Culture Method

Meanwhile, the "third culture method" for a cell population containing Tie2-positive stem/progenitor cells according to the invention is a method of culturing a cell population containing Tie2-positive stem/progenitor cells by using cultureware with a culture surface having undergone cell attachment-increasing treatment.

Conventionally, like many other stem/progenitor cells, a cell population containing Tie2-positive cells (nucleus pulposus stem/progenitor cells) derived from an intervertebral disc nucleus pulposus tissue has also been cultured so as to form floating spheroid colonies under "cell attachment-interfering conditions" as described in Patent Document 1 above, that is, by using low-adhesion cultureware or using a methylcellulose culture medium. However, the present inventors have found that the nucleus pulposus stem/progenitor cells, preferably the cell population containing the nucleus pulposus stem/progenitor cells in which the expression of Tie2 is enhanced by the first culture method and/or the second culture method as described above, can be cultured by being attached to their culture surface without forming spheroid colonies of the nucleus pulposus stem/progenitor cells in a two-dimensional culture environment (without using, for instance, methylcellulose) using "cultureware with a culture surface having undergone cell attachment-increasing treatment (cell attachment treatment)", for example, cultureware coated with a coating agent containing polylysine, rather than "under cell attachment-interfering conditions".

Such a third culture method may be implemented in a step at the differentiation culture stage to increase the efficiency of differentiation from nucleus pulposus stem/progenitor cells into functional nucleus pulposus cells (e.g., Col2-positive cells) more than in the case of using cultureware without culture surface treatment (or, instead, cultureware having undergone cell attachment-inhibiting treatment (low-adhesion treatment). This makes it possible to prepare a cell population having an increased number or ratio of Col2-positive cells in the cell population.

Note that the third culture method may also be performed in a culturing step at the amplification culture stage as a method fused with the second culture method described above. That is, when the Tie2-positive stem/progenitor cells are cultured using cultureware having undergone cell attachment treatment in a culture medium containing a Tie2 activator, the Tie2-positive stem/progenitor cells can be efficiently amplified under a two-dimensional culture-like environment (without using, for instance, methylcellulose).

### Fourth Culture Method

The "fourth culture method" for a cell population containing Tie2-positive stem/progenitor cells according to the invention is a method of culturing a cell population containing Tie2-positive stem/progenitor cells while suppressing formation of spheroid colonies in a culture medium containing an extracellular matrix-degrading agent.

Conventionally, as described above in relation to the first culture method, a substance having an action of degrading an extracellular matrix (e.g., collagen, proteoglycan), such as collagenase or another protease, has been usually used for separating cells from a tissue collected, or temporarily used in a culture method in which spheroid colonies formed by stem/progenitor cells are dissociated once, the culture medium is replaced, and spheroid colonies are formed again.

However, the present inventors have found that a substance having an action of degrading an extracellular matrix (extracellular matrix-degrading agent) such as collagenase can be utilized for completely different purposes (for applications). That is, the present inventors have found that, in the case of Tie2-positive stem/progenitor cells such as nucleus pulposus stem/progenitor cells capable of differentiating into nucleus pulposus cells (more preferably, those in which the expression of Tie2 is enhanced by the first culture method and/or the second culture method), the cells, surprisingly, can be cultured even in a culture medium containing an extracellular matrix-degrading agent, that is, in a situation where spheroid colonies, in which Tie2-positive stem/progenitor cells are bound to each other via an extracellular matrix, cannot be formed, and further, the cells while proliferating can be differentiated into cells positive for expression of extracellular matrices such as type II collagen and proteoglycan.

The present inventors have further found that the above-described "third culture method" and "fourth culture method" may be combined, preferably fused while the type and concentration of the extracellular matrix-degrading agent in the culture medium and the kind of the coating agent on the cultureware surface are appropriately combined to synergistically and markedly increase the efficiency of differentiation from nucleus pulposus stem/progenitor cells into functional nucleus pulposus cells such as type II collagen (Col2)-positive cells, thereby capable of preparing a cell population having a significantly higher number or ratio of Col2-positive cells than in a conventional method.

Moreover, the present inventors have also found that the third culture method and/or the fourth culture method may be used to obtain, at the stage where the number or ratio of, for instance, Col2-positive cells reaches a certain level, a cell population having a certain level of the number or ratio thereof while the Tie2-positive stem/progenitor cells do not completely disappear. Such a cell population contains a certain level of the number or ratio of Tie2-positive stem/progenitor cells, and is thus useful because the therapeutic effects on an intervertebral disc nucleus pulposus when the cell population is administered, for instance, are better.

Based on each culture method described above, the present inventors have established a method of preparing, from a cell population containing Tie2-positive stem/progenitor cells (e.g., nucleus pulposus stem/progenitor cells), a cell population containing target cells (e.g., Col2-positive nucleus pulposus cells) differentiated from the Tie2-positive stem/progenitor cells. This method of preparing a cell population includes at least one and preferably both of a culture stage (amplification culture stage) for amplifying Tie2-positive stem/progenitor cells in the cell population by amplifying the Tie2-positive stem/progenitor cells while enhancing the expression of Tie2, and/or a culture stage (differentiation culture stage) for inducing differentiation from the Tie2-positive stem/progenitor cells into target cells. The amplification culture stage includes a step of performing at least one and preferably both of the first culture method and/or the second culture method (these methods may be fused). The differentiation culture stage includes a step of performing at least one and preferably both of the third culture method and/or the fourth culture method (these methods may be fused). The cell population-preparing method comprising the amplification culture stage including a step of performing both the first culture method and the second culture method (preferably as a fused method) and the differentiation culture stage including a step of performing both the third culture method and the fourth culture method (preferably as a fused method) is an excellent embodiment in the invention. This method may be used to prepare a cell population having a markedly higher number or ratio of target cells having a given functionality than that in a conventionally known preparation method. In the conventional preparation method, the total number of cells in the cell population or the number of the nucleus pulposus cells satisfies a certain level. However, there are not so many functional nucleus pulposus cells, for instance, positive for expression of Col2, among them. In another method, although the ratio of Col2-positive cells in the cell population and the level of expression in individual cells satisfy a certain level, the absolute number of Col2-positive cells is insufficient (i.e., the number of Col2-positive cells amplified from a nucleus pulposus tissue that can be collected from one donor is limited) in this situation. As described above, it has been difficult to achieve both the number of Col2-positive cells and the level of expression (intensity of expression) in the cell population involving a nucleus pulposus. However, the preparation method of the invention can be said to be an innovative preparation method that has not been proposed so far and in which both the number and the level have been successfully achieved.

From another point of view, in order to take advantage of the property that (Tie2-positive) stem/progenitor cells have a potential of anchorage-independent growth and are capable of forming spheroid colonies, or in order to avoid losing any desired functionality by culturing cells differentiated from the stem/progenitor cells on a culture surface (scaffold), a method has conventionally been adopted in which while a cell population contained in a tissue collected is cultured in a methylcellulose culture medium or on a low-adhesion culture surface, the stem/progenitor cells are differentiated into target cells having a given functionality. It can be said that the present inventors have found, by each of the above-described culture methods (particularly, the third culture method and the fourth culture method), an innovative method capable of significantly enhancing the efficiency of proliferation of (Tie2-positive) stem/progenitor cells and differentiation from the stem/progenitor cells into target cells having a given functionality without using methylcellulose, which makes recovery of cells difficult due to high viscosity, and under an environment allowing the cells to adhere to the culture surface so at to recover the produced cell population from the culture medium easily and without waste.

In connection with the culture methods and preparation methods described above, particularly in connection with the first culture method and the amplification culturing step, the present inventors have found a preferable method of preserving a cell population containing Tie2-positive stem/progenitor cells, which method may be used to maintain a state in which Tie2 is activated and/or expressed or to suppress a decrease in Tie2-positive stem/progenitor cells in the cell population, by cryopreserving the cell population containing Tie2-positive stem/progenitor cells while present in a non-digested tissue. Conventionally, a cell population containing Tie2-positive stem/progenitor cells included in a disc nucleus pulposus tissue collected is separated from the tissue by digestion treatment using, for instance, collagenase, which treatment takes a relatively long time, and just the cell population is then cryopreserved. However, immediate cryopreservation of the collected disc nucleus pulposus tissue makes it possible to improve utility during the working process and maintain the cell population while a favorable niche in the tissue (particularly collected from a young donor) is preserved. After such a cryopreserved tissue is thawed, the thawed tissue may be placed in a culture medium to culture the cell population by the first culture method described above, thereby capable of efficiently amplifying the Tie2-positive stem/progenitor cells.

If the above-described technical idea is embodied in combination with a (preferred) embodiment(s) described later in detail, the invention can be expressed, for example, as an invention encompassing at least the following items.

[1] A method of culturing a cell population containing stem cells and/or progenitor cells positive for expression of Tie2 (tyrosine kinase with Ig and EGF homology domain-2) (hereinafter referred to as "Tie2-positive stem/progenitor cells"), the method comprising:
   culturing the cell population containing Tie2-positive stem/progenitor cells while present in a non-digested tissue (hereinafter, the method is referred to as a "first culture method").
[2] The first culture method according to item 1, wherein the Tie2-positive stem/progenitor cells are Tie2-positive stem/progenitor cells derived from a nucleus pulposus (nucleus pulposus) tissue of an intervertebral disc.
[3] The first culture method according to item 1 or 2, wherein the non-digested tissue is a nucleus pulposus tissue of an intervertebral disc.
[4] The first culture method according to any one of items 1 to 3, wherein the non-digested tissue is a tissue obtained by thawing a cryopreserved tissue.
[5] The first culture method according to any one of items 1 to 4, which is performed while the Tie2-positive stem/progenitor cells in the cell population are amplified.
[6]
   A method of culturing a cell population containing Tie2-positive stem/progenitor cells, the method comprising:
   culturing the cell population containing Tie2-positive stem/progenitor cells in a culture medium containing at least one kind of Tie2 expression enhancer other than growth factors (hereinafter, the method is referred to as a "second culture method").
[7] The second culture method according to item 6, wherein the Tie2 expression enhancer other than growth factors is an animal/plant-derived extract.
[8] The second culture method according to item 7, wherein the plant is a plant of the genus *Cinnamomum.*
[9] The second culture method according to any one of items 6 to 8, which is performed while the Tie2-positive stem/progenitor cells in the cell population are amplified.
[10]
   A method of culturing a cell population containing Tie2-positive stem/progenitor cells, the method comprising:
   culturing the cell population containing Tie2-positive stem/progenitor cells by using cultureware with a culture surface having undergone cell attachment-increasing treatment (hereinafter, the method is referred to as a "third culture method").
[11] The third culture method according to item 10, wherein the Tie2-positive stem/progenitor cells have undergone Tie2 expression-enhancing treatment.
[12] The third culture method according to item 10 or 11, wherein the cell attachment-increasing treatment is treatment of applying a coating agent containing an extracellular matrix and/or a polyamino acid.
[13] The third culture method according to any one of items 10 to 12, which is performed while the Tie2-positive stem/progenitor cells in the cell population are differentiated into target cells.
[14] The third culture method according to item 12 or 13, wherein the extracellular matrix and/or the polyamino acid is at least one kind selected from the group consisting of type IV collagen, fibronectin, and polylysine.
[15] The third culture method according to any one of items 10 to 14, which is performed while the Tie2-positive stem/progenitor cells in the cell population are amplified.
[16] The third culture method according to any one of items 12 to 15, wherein the extracellular matrix is gelatin.
[17]
   A method of culturing a cell population containing Tie2-positive stem/progenitor cells, the method comprising:
   culturing the cell population containing Tie2-positive stem/progenitor cells while suppressing formation of spheroid colonies in a culture medium containing an extracellular matrix-degrading agent (hereinafter, the method is referred to as a "fourth culture method").
[18] The fourth culture method according to item 17, wherein the Tie2-positive stem/progenitor cells have undergone Tie2 expression-enhancing treatment.
[19] The fourth culture method according to item 17 or 18, wherein the extracellular matrix-degrading agent comprises at least a protease having activity to degrade type II collagen.
[20] The fourth culture method according to any one of items 17 to 19, which is performed while the Tie2-positive stem/progenitor cells in the cell population are differentiated into target cells.
[21]
   A method of preparing a cell population containing Tie2-positive stem/progenitor cells, the method comprising:
   a culture stage (hereinafter, referred to as an "amplification culture stage") comprising a step of performing the first culture method according to item 5 and/or the second culture method according to item 9 to enhance expression of Tie2 in the Tie2-positive stem/progenitor cells and amplify the Tie2-positive stem/progenitor cells in the cell population.
[22] The preparation method according to item 21, wherein the step performed at the amplification culture stage is a step of simultaneously performing the first culture method and the second culture method.
[23] The preparation method according to item 21 or 22, wherein the amplification culture stage further comprises a step of culturing the cell population containing the Tie2-positive stem/progenitor cells in a culture medium only containing, as a Tie2 expression enhancer, a growth factor having a Tie2 expression-enhancing effect.
[24]
   A method for preparing, from a cell population containing Tie2-positive stem/progenitor cells, a cell population containing target cells differentiated from the Tie2-positive stem/progenitor cells, the method comprising:
   a culture stage (hereinafter referred to as a "differentiation culture stage") comprising a step of performing the third culture method according to item 13 or 14 and/or the fourth culture method according to item 20 to induce differentiation from the Tie2-positive stem/progenitor cells into the target cells.
[25] The preparation method according to item 24, wherein the step performed at the differentiation culture stage is a step of simultaneously performing the third culture method and the fourth culture method.
[26] The preparation method according to item 24 or 25, wherein the target cells are cells expressing at least type II collagen.
[27] The preparation method according to item 26, wherein the cells expressing at least type II collagen are nucleus pulposus cells.
[28] The preparation method according to any one of items 24 to 27, wherein a cell population in which the Tie2-positive stem/progenitor cells remain is obtained through the differentiation culture stage.
[29] A method for preparing, from a cell population containing Tie2-positive stem/progenitor cells, a cell population containing target cells differentiated from the Tie2-positive stem/progenitor cells, the method comprising:
   the amplification culture stage according to any one of items 21 to 23; and
   the differentiation culture stage according to any one of items 24 to 28.
[30]
   A cell population obtained by the culture method according to any one of items 1 to 20.
[31] A culture comprising a culture medium in the culture method according to any one of items 1 to 20 and a cell population to be subjected to the culture method, being cultured, or produced.
[32] A cell population obtained through the amplification culture stage and/or the differentiation culture stage in the preparation method according to any one of items 21 to 29.
[33] A culture comprising a culture medium for amplification culture stage or a culture medium for differentiation culture stage and a cell population to be subjected to the amplification culture stage or the differentiation culture stage, respectively, being cultured, or produced in the preparation method according to any one of items 21 to 29.
[34]
   A composition for cell therapy, comprising the cell population according to item 30 or 32.
[35] The composition for cell therapy according to item 34 for use in treatment or prevention of a disease having a disorder, degeneration, or herniation of an intervertebral disc as a manifested symptom.
[36]
   A method of preserving a cell population containing Tie2-positive stem/progenitor cells, the method comprising:
   cryopreserving a cell population containing Tie2-positive stem/progenitor cells while present in a non-digested tissue to maintain a state in which Tie2 is activated and/or expressed or to suppress a decrease in the Tie2-positive stem/progenitor cells in the cell population.

### [Advantages of the Invention]

A target cell-rich cell population can be prepared by the methods of culturing Tie2-positive stem/progenitor cells according to the invention, preferably, the culture method including an amplification culturing step mainly aimed at amplifying Tie2-positive stem/progenitor cells and a differentiation culturing step mainly aimed at inducing differentiation from the Tie2-positive stem/progenitor cells into mature cells having a given phenotype. Such a cell population obtained based on the culture method(s) of the present invention may be used to efficiently produce a cell preparation effective for treatment or prevention of a predetermined disease.

In addition, in the invention, it is unnecessary to add, to a culture medium, a highly viscous component such as methylcellulose used in the prior art described in, for instance, Patent Documents 1 and 2. This makes it possible to recover, from a culture medium without waste, a cell population containing Tie2-positive stem/progenitor cells or target cells differentiated therefrom.

According to a representative embodiment of the invention, an intervertebral disc (nucleus pulposus) that can be collected only in a small amount from a patient with disc herniation by surgery is used to efficiently amplify and differentiate Tie2-positive stem/progenitor cells (e.g., nucleus pulposus stem cells) contained therein. Accordingly, a suitable cell population that should elicit a high therapeutic effect upon implantation, that is, a cell population rich in functional nucleus pulposus cells having increased production of extracellular matrix such as type II collagen (and some remaining Tie2-positive stem/progenitor cells) can be easily, efficiently, and reproducibly obtained in large amounts. Conventionally, it has been impossible or difficult to produce such a suitable cell population. Since the invention makes that possible, regeneration therapy of an intervertebral disc by administration of (a cell preparation containing) such a cell population is dramatically facilitated, and industrialization becomes realistic.

It is hypothesized that the reasons why the effects of the fourth culture method of the invention are exerted are because the principle shown in Fig. 1, for instance, is working. However, this hypothesis is intended to aid understanding of the invention, and the invention is not necessarily bound thereby. Even if it is found afterwards that some or all of the effects of the invention are exerted based on a principle and/or a mechanism of action different from those illustrated in Fig. 1, the effects of the invention that can be actually found and elements of the invention therefor should not be denied by the following description based on Fig. 1.

Fig. 1[A] shows how cultured cells look when a cell population containing Tie2-positive stem/progenitor cells (e.g., nucleus pulposus stem/progenitor cells) is amplified and differentiated by two-dimensional culture (monolayer static culture). The culture surface of cultureware (e.g., a flask) may be subjected to cell attachment-enabling surface treatment of, for instance, applying a coating agent containing an extracellular matrix (ECM) in advance. The stem/progenitor cells adhere to the culture surface of cultureware and differentiate while spreading and growing on the culture surface. In such two-dimensional culture, intracellular signaling that eventually stops the production and secretion of ECM occurs due to the interaction between the ECM, which has been applied on the culture surface or secreted from the cultured cells, and the binding protein(s) (e.g., integrin), which is expressed on a surface of the cultured cells. For example, in the case of two-dimensionally culturing a nucleus pulposus derived cell population, ECM such as type II collagen and proteoglycan is actively secreted from mature nucleus pulposus cells originally contained in the cell population and mature nucleus pulposus cells generated while nucleus pulposus stem/progenitor cells contained in the cell population are differentiated while proliferating. However, as the culture period is elapsed, the production and secretion of, for instance, type II collagen are eventually stopped by the intracellular signaling as described above (instead, the production and secretion of type I collagen increases). Then, dedifferentiation of mature nucleus pulposus cells occurs to exhibit, for instance, a fibroblast-like phenotype. Thus, in typical two-dimensional culture, it is considered to be difficult to achieve both an increase in the number of cells in the cell population and an increase in the proportion of cells having a specific phenotype (cells not dedifferentiated). Note that in the third culture method of the invention, preferably, use of Tie2-positive stem/progenitor cells having increased expression of Tie2 makes it possible to relatively easily prepare a cell population containing a certain proportion of nucleus pulposus cells expressing, for instance, type II collagen even in two-dimensional culture.

Figs. 1[B] and [C] show how cultured cells look when a cell population containing Tie2-positive stem/progenitor cells (e.g., nucleus pulposus stem/progenitor cells) is amplified and differentiated by culture using a methylcellulose-containing culture medium (free of an extracellular matrix (ECM)-degrading agent) or low-adsorption cultureware. Unlike the two-dimensional culture of Fig. 1[A], in the culture as shown in Fig. 1[B], the interaction between the ECM, for instance, on the culture surface of cultureware and the cultured cells does not occur, and intracellular signaling that stops the production and secretion of the ECM due to the interaction also does not occur. Thus, at an early stage of culture, Tie2-positive stem/progenitor cells proliferate while producing and secreting ECM, and eventually form spheroid colonies. However, in the spheroid colonies formed, Tie2-positive stem/progenitor cells or cells differentiated therefrom (e.g., nucleus pulposus cells) come into contact with one another via the secreted ECM. Accordingly, as shown in Fig. 1[C], the interaction between the ECM and the cultured cells occurs as the culture period is elapsed, and an ECM production stop signal is generated by the interaction, and dedifferentiation similar to that in Fig. 1[A] is induced. Thus, in the culture method as shown in Figs. 1[B] and [C], it is difficult to increase the percentage of cells positive for expression of a given ECM (e.g., type II collagen) to a certain level or more in the cell population recovered as spheroid colonies.

Fig. 1[D] shows how Tie2-positive cultured cells look when a cell population containing Tie2-positive stem/progenitor cells is amplified and differentiated by culture using a culture medium (free of, for instance, methylcellulose) containing an extracellular matrix (ECM)-degrading agent according to the fourth culture method of the invention. Even in such a culture method, like in Fig. 1[B], ECM is secreted from stem/progenitor cells or cells differentiated therefrom. However, the ECM secreted extracellularly is constantly degraded by the ECM-degrading agent added to the culture medium. Due to this, neither spherical colonies are formed, nor cells attach to the culture surface even without using low-adsorption cultureware. Further, in the third-fourth culture method of the invention, like in the two-dimensional culture of Fig. 1[A], attachment of cells to the culture surface remains weak even if a coating agent containing ECM is applied beforehand onto the culture surface of the cultureware. Thus, the ECM production stop signal caused by the interaction between the ECM and the cultured cells is suppressed, and dedifferentiation as in Fig. 1[A] or Figs. 1[B] and [C] is less likely to occur. This makes it possible to prepare a cell population in which the percentage of cells positive for expression of a predetermined ECM (e.g., type II collagen) is increased more than in the conventional technology.

Note that the ECM secreted extracellularly is degraded by the ECM-degrading agent in the culture medium, but intracellular ECM is not degraded and is progressively accumulated. Here, the fourth culture method of the invention may be performed in a step at the differentiation culture stage, and the resulting cell population may then be recovered from the culture medium to produce a cell preparation. In this case, intracellularly accumulated ECM is rapidly secreted extracellularly in the tissue having received the cell preparation. This makes it possible to create an environment fit for survival of the cell population. Therefore, the following ECM production and secretion from the administered cells (i.e., therapeutic effects exerted by the cell preparation) should be able to be promoted.

### [Brief Description of Drawings]

Fig. 1 is diagrams schematically illustrating production and degradation of ECM and an interaction between cultured cells in each of regular two-dimensional culture (monolayer static culture), suspension culture using a conventional culture medium free of an extracellular matrix (ECM)-degrading agent, or suspension culture using a culture medium containing an ECM-degrading agent according to the fourth culture method of the invention, and photographs of respective cultured cells. A (Regular two-dimensional culture): when nucleus pulposus (nucleus pulposus) cells adhere via an adhesion molecule(s) to the culture surface of a culture flask, an ECM production stop signal is transduced. B (Suspension culture using, for instance, a low-adsorption flask, methylcellulose culture medium): because of no contact with the flask culture surface, no ECM production stop signal is transduced (× mark and the dotted arrow). C (Culture medium without an enzyme): the self-produced ECM exhibits an action equivalent to that of the culture surface (the arrows), and an ECM production stop signal is transduced into the cells. D (Culture medium with an enzyme): The self-produced ECM is degraded, and no ECM production stop signal is transduced into the cells (× mark and the dotted arrow), but accumulation of ECM in cells occurs.
Fig. 2 is a graph showing the results of the Tie2-positive rate in Test Example 1 (the amplification culture stage: the first culturing step).
Fig. 3 is a graph showing the results of Tie2 mean fluorescence intensity (MFI) in Test Example 1 (the amplification culture stage: the first culturing step).
Fig. 4 is a graph showing the results of the Tie2-positive rate in Test Example 2 (the amplification culture stage (two steps): the first-second culturing step + an additional step).
Fig. 5 is a graph showing the results of the number of Tie2-positive cells produced per g of a nucleus pulposus tissue in Test Example 2 (the amplification culture stage (two steps): the first-second culturing step + an additional step).
Fig. 6 is a graph showing the results of the type II collagen (Col2)-positive rate in Test Example 3 (the amplification culture stage (two steps): the first-second culturing step + an additional step -> the differentiation culture stage: the third culturing step).
Fig. 7 is a graph showing the results of the number of type II collagen (Col2)-positive cells produced per g of a nucleus pulposus tissue in Test Example 3 (the amplification culture stage (two steps): the first-second culturing step + an additional step -> the differentiation culture stage: the third culturing step).
Fig. 8 is a graph showing the results of the proteoglycan (PG)-positive rate in Test Example 4 (the amplification culture stage (two steps): the first-second culturing step + an additional step -> the differentiation culture stage: the third-fourth culturing step).
Fig. 9 is a graph showing the results of the type II collagen (Col2)-positive rate in Test Example 4 (the amplification culture stage (two steps): the first-second culturing step + an additional step -> the differentiation culture stage: the third-fourth culturing step).
Fig. 10 is graphs showing the results of the proteoglycans (PG)- or collagen II (Col2)-positive rate in Test Example 5 (the amplification culture stage (two steps): the first-second culturing step + an additional step -> the differentiation culture stage: the third-fourth culturing step; part 2). GEL: gelatin, Coll: type I collagen, Col4: type IV collagen, FN: fibronectin, PLL: poly-L-lysine (the same applies to Fig. 11).
Fig. 11 is graphs showing the results of the proteoglycans (PG)- or collagen II (Col2)-positive rate in Test Example 6 (the amplification culture stage (two steps): the first-second culturing step + an additional step -> the differentiation culture stage: the third-fourth culturing step; part 3).

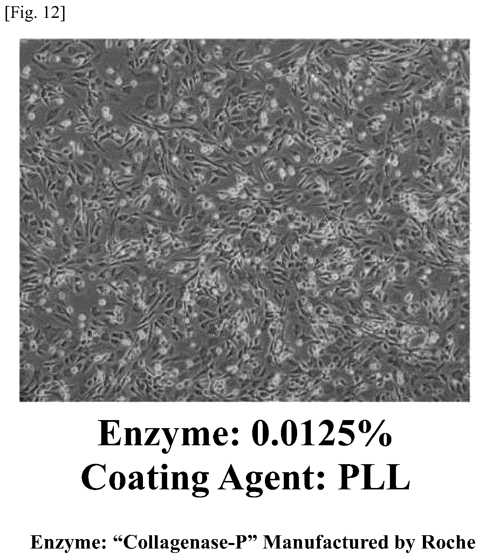

Fig. 12 is an optical micrograph of a cell population in Test Example 5-12.
Fig. 13 is a graph showing the results of the Tie2-positive rate in Test Example 7 (the differentiation culture stage: the third culturing step).
Fig. 14 is a graph showing the results of the total number of Tie2-positive cells in Test Example 7 (the differentiation culture stage: the third culturing step).
Fig. 15 is a graph showing the results of the Col2-positive rate in Test Example 7 (the differentiation culture stage: the third culturing step).

### [Mode for Carrying Out the Invention]

### -Terms-

The term "stem cell(s)" refers to a cell(s) having self-renewal ability and differentiation potential (i.e., totipotent, pluripotent, multipotent, or unipotent cells). The term "progenitor cell(s)" refers to a cell(s) without self-renewal ability in a strict sense because all of the cells finally become terminally differentiated cells, but with some differentiation potential to differentiate into a predetermined cell(s) while relatively actively proliferating. (Identified) cells generally understood and called, by those skilled in the art, as "stem cells" or "progenitor cells" herein correspond to "stem cells" or "progenitor cells".

As used herein, the wording "stem cells and/or progenitor cells" includes stem cells, progenitor cells, or both, and is sometimes referred to as "stem/progenitor cells". In addition, as used herein, a cell population containing stem cells and/or progenitor cells may be referred to as a "stem/progenitor cell population", and a cell population containing mature cells differentiated from the stem cells and/or progenitor cells (i.e., terminally differentiated cells) may be referred to as a "mature cell population".

In general, the "stem cells" and the "progenitor cells" are distinguishable from other cells by whether the expression of one or two or more kinds of specific genes (marker genes or cell markers) is positive or negative. That is, the "stem cells" having self-renewal ability and/or differentiation potential as described above or the "progenitor cells" can also be defined as terms that refer to cells in which the expression of a specific marker gene is positive or negative, respectively.

Whether the expression of a marker gene (cell marker) is "positive" or "negative" can be determined by quantitatively or qualitatively measuring the expression level of mRNA transcribed from the gene (genome) or protein translated from the mRNA according to a common protocol. If the expression level is a certain level or higher (or higher than a certain level), the expression can be determined to be positive, and if the expression level is a certain level or lower (or lower than a certain level), the expression can be determined to be negative. The expression level of a protein can be measured quantitatively or qualitatively by an immunological assay (e.g., flow cytometry, immunostaining, or ELISA) using, for instance, an antibody or labeling agent specific to the protein. Note that the Tie2 protein is a protein expressed on the cell surface, and Col2 is a protein expressed inside a cell. Appropriate techniques (e.g., immunofluorescence staining) may be used to detect proteins present on the cell surface or inside a cell, respectively. The expression level of mRNA can be measured quantitatively or qualitatively by, for example, an assay (e.g., RT-PCR, a microarray, or a biochip) using a nucleic acid and a labeling agent or an amplification protocol (means) for nucleic acid specific (complementary) to the mRNA. The percentage (positive rate or negative rate) of cells positive or negative for expression of a given marker gene (cell marker) in a cell population can be calculated by counting the number of all cells in the cell population and the number of cells determined to be positive or negative by the above-described protocols, respectively, while using the above various techniques such as flow cytometry.

As used herein, the wording "stem cells and/or progenitor cells positive for expression of Tie2", that is, "Tie2-positive stem/progenitor cells" refers to cells characteristic of stem cells and/or progenitor cells, in which expression of Tie2 (tyrosine kinase with Ig and EGF homology domain-2) known as one of the cell markers, for example, its expression as a protein measured by flow cytometry is determined to be positive. Representative Tie2-positive stem/progenitor cells in the invention are Tie2-positive stem/progenitor cells "derived from a nucleus pulposus tissue of an intervertebral disc", that is, Tie2-positive stem/progenitor cells present in the nucleus pulposus of a intervertebral disc (harvested from the nucleus pulposus) or Tie2-positive stem/progenitor cells obtained by subculturing the Tie2-positive stem/progenitor cells, and are cells corresponding to "nucleus pulposus stem/progenitor cells" described below.

As used herein, the term "target cells" refers to cells obtained from Tie2-positive stem/progenitor cells by inducing differentiation in a given manner and having a functionality according to their use, more specifically, cells in which expression of a given gene (cell marker) is determined to be positive or negative for expression as a protein, for example, by flow cytometry. Typical target cells in the invention are among "nucleus pulposus cells" described below and positive for expression of genes of extracellular matrix (ECM) such as Col2 and aggrecan.

The "nucleus pulposus cells" in the invention refer to matured and terminally differentiated cells that account for majority of cells in a population in an intervertebral disc (nucleus pulposus), or cultured cells having an equivalent phenotype.
Specifically, the nucleus pulposus cells can be defined as cells negative for Tie2 and GD2 as marker genes (in addition, usually positive for CD 24), and positive for at least type II collagen among extracellular matrix proteins (in addition, usually also positive for proteoglycan (aggrecan)). For example, cells determined to be negative for Tie2 and GD2 (and positive for CD 24) and positive for type II collagen (and also positive for aggrecan) as proteins (cell markers) by flow cytometry correspond to nucleus pulposus cells in the invention. For extracellular matrices such as type II collagen and aggrecan, the amount of each protein produced may be measured by flow cytometry, and the expression level of each mRNA may be measured by, for instance, real-time PCR.

As used herein, the term "nucleus pulposus stem/progenitor cells" collectively refers to progenitor cells (nucleus pulposus progenitor cells) having at least a potential to differentiate into nucleus pulposus cells and stem cells (nucleus pulposus stem cells) having self-renewal ability and a potential to differentiate into the progenitor cells, or cultured cells having an equivalent phenotype, which cells account for part of a cell population in a nucleus pulposus tissue of an intervertebral disc. The nucleus pulposus stem/progenitor cells may be specifically defined as cells that are positive for Tie2 and/or GD2 as marker genes. For example, cells determined to be either positive for Tie2 and negative for GD2, positive for Tie2 and positive for GD2, or negative for Tie2 and positive for GD2 as proteins (cell markers) by flow cytometry correspond to the nucleus pulposus stem/progenitor cells in the invention.

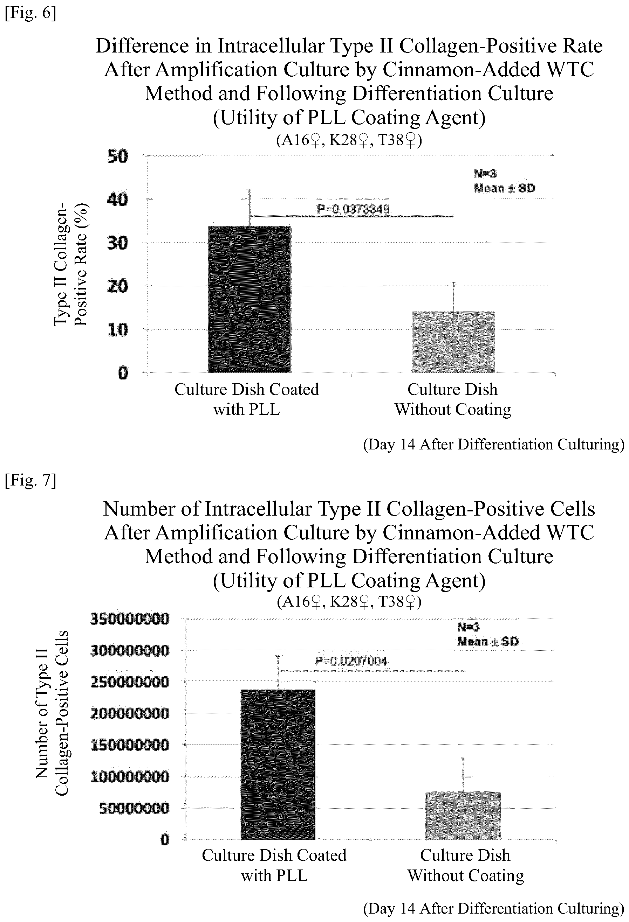

Note that, in Patent Document 2 described above, cells positive for Tie2 are classified into "disc nucleus pulposus stem cells" (among them, cells negative for GD2 are in a dormant state, and cells positive for GD2 are in an active state); cells negative for Tie2 and positive for GD2 are classified into "disc progenitor cells"; and cells negative for Tie2 and negative for GD2 are classified into "terminally differentiated mature disc nucleus pulposus cells", based on the expression states of Tie2 and GD2 as cell markers of nucleus pulposus-derived cells (paragraphs [0024], [0025], and [0032]). In addition, in Patent Literature 2, cells appearing in the differentiation hierarchy of nucleus pulposus cells are grouped into: (i) cells that are Tie2⁺ and GD2⁻ (further, CD24⁻, CD44^{+/-}, CD271⁺, Flt1⁺); (ii) cells that are Tie2⁺ and GD2⁺ (further, CD24⁻, CD44⁺, CD271⁺, Flt1⁺); (iii) cells that are Tie2⁻ and GD2⁺ (further, CD24⁻, CD44⁺, CD271^{+/-}, Flt1^{+/-}); (iv) cells that are Tie2⁻ and GD2⁺ (further, CD24⁺, CD44⁺, CD271⁻, Flt1⁻); or (v) cells that are Tie2⁻ and GD2⁻ (further, CD24⁺, CD44⁺, CD271⁻, Flt1⁻). The above groups (i) to (iii) are termed as "disc nucleus pulposus stem/progenitor cells" and the above groups (iii) to (v) are termed as "nucleus pulposus committed cells" (see Fig. 7-2). Although the terminology is different, the "disc nucleus pulposus stem cells" and the "disc nucleus pulposus progenitor cells" of Patent Document 2, that is, the cells of the above groups (i) to (iv) correspond to the "nucleus pulposus stem/progenitor cells" in the invention; and the "terminally differentiated mature disc nucleus pulposus cells" of Patent Document 2, that is, the cells of the above group (v) correspond to the "mature nucleus pulposus cells" in the invention. If necessary, the cells in the invention may be replaced by cells according to the definition described in Patent Document 2 (in particular, the definition of whether one or two or more kinds of cell markers, such as CD24, other than Tie2 and GD2 are positive or negative).

The "spheroid colony" herein refers to a spherical cell aggregate which contains stem cells and/or progenitor cells and further optionally contains cells differentiated therefrom. The "spheroid colony" is an object that may be generally referred to as, for instance, a "sphere" or "spheroid" by those skilled in the art, and the "discosphere" or "free floating circular-spherical structure" in Patent Document 2 described above is also an object corresponding to the "spheroid colony".

As used herein, the wording "expression of Tie2 is enhanced" (enhanced expression of Tie2) means that the expression of Tie2 gene is enhanced in individual stem/progenitor cells, that is, the expression is augmented more than usual, and the expression level of mRNA or protein is increased. Even under regular conditions in which the expression of Tie2 gene almost disappears, the wording "expression of Tie2 is enhanced" corresponds to keeping a certain level of expression without loss of expression, that is, maintaining the expression of Tie2. In addition, as a result of such enhanced expression of Tie2 in individual stem/progenitor cells, an increase in the number of cells determined to have positive expression of Tie2 mRNA or protein in the cell population, that is, a higher percentage of Tie2-positive cells in the cell population than usual can also be understood as an indicator of "enhanced Tie2 expression".

More specifically, for example, Tie2 protein on the cell surface is fluorescently labeled for a cell population that has previously undergone Tie2 expression-enhancing treatment (Tie2 expression-enhancing treatment group) or a cell population that has not undergone Tie2 expression-enhancing treatment (control group). When measured by flow cytometry, the percentage of cells determined to have a higher fluorescence intensity and more positive expression and/or a higher average fluorescence intensity per cell than a predetermined level may be higher in the Tie2 expression-enhancing treatment group than in the control group. In this case, it can be said that (Tie2-expressing cells contained in) the cell population of the Tie2 expression-enhancing treatment group has enhanced expression of Tie2 (in other words, the Tie2 expression-enhancing treatment plays a prescribed role). Further, under morphological observation, cells having enhanced expression of Tie2 are also distinguishable by having a spindle shape (the rest cells have a near-spherical shape).

An agent that exerts the effects of "enhancing Tie2 expression" as described above is herein referred to as a "Tie2 expression enhancer" in the invention. Note that some growth factors (e.g., FGF2) have a Tie2 expression-enhancing effect, and can be said to correspond to a kind of "Tie2 expression enhancer". Accordingly, in the case of excluding such growth factors, the agent is called a "Tie2 expression enhancer other than growth factors".

In the invention, performing the first culture method and/or the second culture method corresponds to subjecting a cell population containing Tie2-positive stem/progenitor cells to "Tie2 expression-enhancing treatment".

### -Culture Methods-

The first to fourth culture methods for a cell population containing Tie2-positive stem/progenitor cells according to the invention are as follows:
the first culture method: a method of culturing a cell population containing Tie2-positive stem/progenitor cells while present in a non-digested tissue;
the second culture method: a method of culturing a cell population containing Tie2-positive stem/progenitor cells in a culture medium containing at least one kind of Tie2 expression enhancer other than growth factors;
the third culture method: a method of culturing a cell population containing Tie2-positive stem/progenitor cells by using cultureware with a culture surface having undergone cell attachment-increasing treatment; and
the fourth culture method: a method of culturing a cell population containing Tie2-positive stem/progenitor cells while suppressing formation of spheroid colonies in a culture medium containing an extracellular matrix-degrading agent.

The first to fourth culture methods of the invention may be implemented singly, or may be implemented sequentially or simultaneously by combining a plurality of the culture methods. A plurality of culture methods selected from the first to fourth culture methods may be combined and performed simultaneously. This means that the selected culture methods are fused, that is, the culture method that meets all the technical matters involving the selected culture methods is carried out. For example, the first culture method and the second culture method may be combined (fused) and performed sequentially or simultaneously (a method in which these methods are fused may be referred to as the "first-second culture method"). The third culture method and the fourth culture method can be combined (fused) and performed sequentially or simultaneously (a method in which these methods are fused may be referred to as the "third-fourth culture method").

The purpose of performing the first to fourth culture methods of the invention is not particularly limited. The first to fourth culture methods may each be performed in any of the amplification culture stage (or a stage corresponding thereto), the differentiation culture stage (or a stage corresponding thereto), or other stages in the invention.

Unless otherwise specified, the description about the first to fourth culture methods (and the first to fourth culturing steps of performing the first to fourth culture methods) may be read, if appropriate, as a description not only in a case where each method is carried out as a single method (step) but also in a case where each method is carried out as a method (step) fused to another method (step).

The Tie2-positive stem/progenitor cells contained in the cell population to which the fourth culture method of the invention is applied and the cells differentiated from the stem/progenitor cells may be cells that bind to each other via the extracellular matrix secreted extracellularly to form spheroid colonies (spheroids) in a regular culture medium free of any extracellular matrix-degrading agent. If the extracellular matrix-degrading agent is added to the culture medium in accordance with the invention, the type of the cells is not particularly limited as long as the cells exert the effects of inhibiting the formation of spheroid colonies (spheroids).

In a representative embodiment of the invention, the cells differentiated from the Tie2-positive stem/progenitor cells are cells that produce and secrete more extracellular matrix than typical cells, for instance, nucleus pulposus cells that are responsible for producing and secreting extracellular matrix in a disc nucleus pulposus tissue. Mature nucleus pulposus cells express at least type II collagen as an extracellular matrix, and also express an extracellular matrix such as proteoglycan (aggrecan). In a preferred embodiment of the invention, the Tie2-positive stem/progenitor cells are differentiated into cells expressing extracellular matrices such as type II collagen and proteoglycan (aggrecan), particularly functional nucleus pulposus cells having a superior expression level (production level) of not only mRNA but also protein of type II collagen.

### -Preparation Method (Culturing Step)-

The method of preparing, from a cell population containing Tie2-positive stem/progenitor cells, a cell population containing target cells differentiated from the Tie2-positive stem/progenitor cells according to the invention comprises the following amplification culture stage and/or differentiation culture stage, preferably both the amplification culture stage and the differentiation culture stage (in the order of the first amplification culture stage and the next differentiation culture stage).

Amplification culture stage: a culturing step of enhancing expression of Tie2 in Tie2-positive stem/progenitor cells and amplifying the Tie2-positive stem/progenitor cells in a cell population; and
differentiation culture stage: a culturing step of inducing differentiation of the Tie2-positive stem/progenitor cells into target cells.

### -Step Involving Amplification Culture Stage

In a preferred embodiment of the invention, the first culture method and the second culture method are performed in a step at the amplification culture stage. Either one of the first culture method or the second culture method may be performed, or both of them may be performed. Both the first culture method and the second culture method may be implemented. In this case, at the amplification culture stage, the step of performing the first culture method (herein referred to as a "first culturing step") and the step of performing the second culture method (herein referred to as a "second culturing step") may be separate steps that are sequentially performed (the first culturing step is performed first, and the second culturing step is performed later). The two culture methods may be provided as a single step (herein referred to as the "first-second culturing step") (in which the first and second culture methods are fused and performed). That is, the step may be a step of culturing a cell population containing Tie2-positive stem/progenitor cells while present in a non-digested tissue and in a Tie2 expression enhancer-containing culture medium.

The "step at the amplification culture stage" is mainly intended to amplify Tie2-positive stem/progenitor cells by culturing under prescribed conditions, and means a step in which the effects therefor are exerted (relatively stronger than other effects). That is, if the number and/or percentage of Tie2-positive stem/progenitor cells is higher in the post-culture cell population than in the pre-culture cell population, the culturing step can be said to be a "step at the amplification culture stage". Here, it is permitted within the limit that the Tie2-positive stem/progenitor cells are differentiated into other cells (target cells).

The expression of Tie2 in individual Tie2-positive stem/progenitor cells (e.g., nucleus pulposus stem/progenitor cells) in the invention is enhanced (including a case where expression of Tie2 is kept). Also, the number and/or the percentage of Tie2-positive stem/progenitor cells contained in the cell population are increased. Such effects can be synergistically augmented. Thus, it is particularly preferable that the first-second culture method is implemented as a step at the amplification culture stage (i.e., the first-second culturing step is performed).

### - Step Involving Differentiation Culture Stage

In a preferred embodiment of the invention, the third culture method and the fourth culture method are performed in a step at the differentiation culture stage. Either one of the third culture method or the fourth culture method may be performed, or both of them may be performed. Here, both the third culture method and the fourth culture method may be performed. In this case, at the differentiation culture stage, the step of performing the third culture method (herein referred to as a "third culturing step") and the step of performing the fourth culture method (herein referred to as a "fourth culturing step") may be separate steps that are sequentially performed. The two culture methods may be provided as a single step (herein referred to as the "third-fourth culturing step") (in which the third and fourth culture methods are fused and performed). That is, the step may be a step of culturing a cell population containing Tie2-positive stem/progenitor cells by using cultureware with a culture surface having undergone cell attachment-increasing treatment and by suppressing formation of spheroid colonies in a culture medium containing an extracellular matrix-degrading agent.

The "step at the differentiation culture stage" is mainly intended to differentiate Tie2-positive stem/progenitor cells into predetermined cells by culturing under prescribed conditions, and means a step in which the effects therefor are exerted (relatively stronger than other effects). That is, if the number and/or the percentage of the target cells are higher in the post-culture cell population than in the pre-culture cell population, the culturing step can be said to be a "step at the differentiation culture stage".

Note that as described above, the step of temporarily treating, in a culture medium containing collagenase, spheroid colonies (spheroids, discospheres, floating spherical structures) described in Patent Document 1 to dissociate them fails to correspond to the fourth culture method of the invention as defined above or the fourth culturing step as a step at the differentiation culture stage. In addition, a method (step) of treating a cell population contained in a collected tissue with, for instance, collagenase for isolation or a method (step) of subjecting cells grown in typical two-dimensional culture to trypsin treatment to dissociate the cells from the culture surface for subculturing also fails to correspond to the fourth culture method of the invention as defined above or the fourth culturing step as a step at the differentiation culture stage.

Given functional cells (e.g., Col2-positive nucleus pulposus cells) differentiated from Tie2-positive stem/progenitor cells (e.g., nucleus pulposus stem/progenitor cells) contained in a cell population in the invention should have an increased number and/or percentage of the cells. On the other hand, it is possible to synergistically increase the effects of, for instance, keeping at a certain level the number and/or the percentage of the Tie2-positive stem/progenitor cells. In view of the above, it is particularly preferable that the third-fourth culture method is implemented as a step at the differentiation culture stage (i.e., the third-fourth culturing step is performed).

The amplification culture stage may further optionally include a step in addition to the first culturing step and/or the second culturing step, which step meets the purpose of the step of amplifying the Tie2-positive stem/progenitor cells. Examples of such a step include a step of culturing a cell population containing Tie2-positive stem/progenitor cells in a culture medium containing, as a Tie2 expression enhancer, only a growth factor having a Tie2 expression-enhancing effect (this step is herein referred to as an "additional amplification culturing step"). Examples of the growth factor having a Tie2 expression-enhancing effect in the additional amplification culturing step include FGF and/or EGF. The additional amplification culturing step is preferably performed after the first culturing step and/or the second culturing step, particularly the first culturing step or the first-second culturing step. Also, in the additional amplification culturing step, it is suitable that the first culture method is not performed, that is, the cell population containing Tie2-positive stem/progenitor cells is not in a state of being present in a non-digested tissue but in a state where the cells are separated by digestion treatment. In the "first culture method" performed in the first culturing step or the first-second culturing step in the invention, a cell population containing Tie2-positive stem/progenitor cells is cultured while present in a non-digested tissue. However, if the culture reaches a certain level, the presence in the tissue may affect the cells. Then, the Tie2-positive stem/progenitor cells are prevented from amplifying (even if the culture period is extended, the Tie2-positive stem/progenitor cells do not amplify). Thus, after the first culturing step or the first-second culturing step, the tissue is digested, the separated cell population is recovered, and the additional amplification step is performed. This enables the Tie2-positive stem/progenitor cells to be further amplified.

### <Cell Population>

A cell population containing Tie2-positive stem/progenitor cells to be subjected to each culture method or each culturing step in the invention (herein generally referred to as a "pre-culture cell population") includes Tie2-positive stem/progenitor cells and the other cells (e.g., cells differentiated from Tie2-positive stem/progenitor cells) basically at any ratio and/or numbers. Further, basically any ratio between the Tie2-positive stem cells and the Tie2-positive progenitor cells is also permitted. The composition of the pre-culture cell population can be adjusted, if appropriate, according to an embodiment of the invention while the effects in each culture method or each culturing step are considered.

The pre-culture cell population may be provided or prepared according to a conventional procedure except for the case of being subjected to the first culture method or the first culturing step. For instance, a cell population included in an *in vivo* collected disc nucleus pulposus tissue may be used as a pre-culture cell population. In this case, the nucleus pulposus tissue was first finely cut with an instrument such as scissors into pieces with a suitable size (e.g., mince with about several-mm cubes). Next, the resulting cells were treated with a protease such as collagenase, dispersed, and optionally filtered, centrifuged, washed, etc. These treatments enable a cell population included in the nucleus pulposus tissue to be isolated and recovered. The resulting cell population may be used as a pre-culture cell population other than those used in the first culture method or the first culturing step.

On the other hand, in the first culture method or the first culturing step in the invention, the above procedure was stopped at a step of finely cutting the nucleus pulposus tissue (the treatment with a protease is not performed). Then, a cell population while included in the finely cut nucleus pulposus tissue is utilized as a pre-culture cell population.

The cell population separated from the tissue or the cell population (a cell population-containing tissue) while included in the tissue prepared as described above may be cryopreserved according to a conventional procedure until being subjected to the next culture method or culturing step. The cryopreserved cell population or tissue may be thawed according to a conventional procedure when the next culture method or culturing step is started. During cryopreservation and thawing, treatments fit for the cell population or tissue may be combined. For example, a cryoprotectant (e.g., DMSO) may be added during cryopreservation, and in this case, the cryoprotectant may be removed under suitable conditions during thawing.

A cell population containing Tie2-positive stem/progenitor cells obtained by each culture method or each culturing step in the invention (herein generally referred to as a "post-culture cell population") includes Tie2-positive stem/progenitor cells and the other cells (e.g., cells differentiated from Tie2-positive stem/progenitor cells) basically at any ratio and/or numbers. Further, basically any ratio between the Tie2-positive stem cells and the Tie2-positive progenitor cells is also permitted. The composition of the post-culture cell population can be adjusted, if appropriate, according to an embodiment of the invention while use of the cell population obtained by each culture method or each culturing step is considered.

The post-culture cell population may be recovered from the culture medium according to a routine procedure and subjected to the next culture method or culturing step, or subjected to another method or step such as preparation of a cell preparation.

A cell population containing Tie2-positive stem/progenitor cells during the process of each culture method or each culturing step in the invention (herein generally referred to as a "in-culture cell population") includes Tie2-positive stem/progenitor cells and the other cells (e.g., cells differentiated from Tie2-positive stem/progenitor cells) basically at any ratio. Further, basically any ratio between the Tie2-positive stem cells and the Tie2-positive progenitor cells is also permitted. The composition of the in-culture cell population is a composition in the process of transition from the pre-culture cell population to the post-culture cell population. For example, the ratio of Tie2-positive stem/progenitor cells with respect to the in-culture cell population (herein referred to as the "Tie2-positive stem/progenitor cell rate") is a number in the range (inclusive) between the Tie2-positive stem/progenitor cell rate in the pre-culture cell population and the Tie2-positive stem/progenitor cell rate in the post-culture cell population. However, the number is permitted to be temporarily out of the range. The composition of the in-culture cell population varies depending on an embodiment of the invention and depending on, for instance, the number of days and the number of passages in each culture method or each culturing step.

The "human or other animal" (donor) from which each cell population is derived can be selected in consideration of, for instance, use of the cell population finally obtained by the method of culturing Tie2-positive stem/progenitor cells according to the invention or use of the cell population obtained by each culture method or each culturing step included in the method. In an exemplary embodiment of the invention, it is possible to prepare a cell population for producing a cell preparation so as to prevent or treat, for instance, a given disease or symptom. In this case, the "human or other animal" is an organism of the same species as a subject (recipient) receiving the cell preparation, and is preferably a human.

### - Cell Population Involving Amplification Culture Stage

A cell population in the invention (herein generally referred to as a "pre-amplification-culture cell population) is to be subjected to the first culture method and/or the second culture method or the first culturing step and/or the second culturing step at the amplification culture stage. The cell population is typically a cell population (primary culture cell population) contained in a tissue (intervertebral disc) collected from the body of a human or other animal or a cell population (subculture cell population) obtained by subculturing the primary culture cell population.

For instance, a cell population included in an intervertebral disc collected from a human may be used as a pre-amplification-culture cell population. In this case, preferred is a cell population included in an intervertebral disc collected from a human in teens or twenties, which intervertebral disc is likely to have, in general, an increased Tie2-positive stem/progenitor cell rate and superior niche. In addition, the pre-amplification-culture cell population is preferably a cell population having a Tie2-positive stem/progenitor cell rate as high as possible, for example, 30% or more, 40% or more, 50% or more, or 60% or more.

Note that in some embodiments, the pre-amplification-culture cell population is not necessarily a cell population contained in a tissue collected from the body of a human or other animal. For example, the cell population may be a cell population containing Tie2-positive stem/progenitor cells obtained by inducing differentiation of pluripotent or multipotent cells, such as iPS cells or ES cells, which have been produced using cells from a human or other animal.

In the invention, use of the cell population obtained by the first culture method and/or the second culture method or use of the cell population obtained by the first culturing step and/or the second culturing step at the amplification culture stage (herein generally referred to as a "post-amplification-culture cell population) is not particularly limited. The composition of the resulting cell population is adjustable, if appropriate, depending on use thereof.

In a typical embodiment of the invention, the post-amplification-culture cell population is used as a cell population to be subjected to the third culture method and/or the fourth culture method, or a cell population to be subjected to the third culturing step and/or the fourth culturing step at the differentiation culture stage. The post-amplification-culture cell population in such an embodiment (use) preferably has a ratio of Tie2-positive stem/progenitor cells and/or the number of the cells as high as possible. The ratio of Tie2-positive stem/progenitor cells in the post-amplification-culture cell population varies depending on, for instance, individual differences of the pre-amplification-culture cell population and the nucleus pulposus tissue from which the pre-amplification-culture cell population is derived. Thus, the ratio depends on the situation, but is, for example, 5% or more, preferably 7% or more, 9% or more, 11% or more, 13% or more, or 15% or more. The number of Tie2-positive stem/progenitor cells in the post-amplification-culture cell population varies depending on, for instance, individual differences of the pre-amplification-culture cell population and the nucleus pulposus tissue from which the pre-amplification-culture cell population is derived. Thus, the ratio depends on the situation, but The number of cells is, for example, 5 times or more, preferably 10 times or more, 15 times or more, 20 times or more, 25 times or more, or 30 times or more the number in the pre-amplification-culture cell population.

### - Cell Population Involving Differentiation Culture Stage

A cell population to be subjected to the third culture method and/or the fourth culture method or a cell population to be subjected to the third culturing step and/or the fourth culturing step at the differentiation culture stage in the invention (herein referred to as a "pre-differentiation-culture cell population") is preferably a cell population obtained by enriching Tie2-positive stem/progenitor cells in advance. The Tie2-positive stem/progenitor cell rate in a pre-differentiation-culture-stage cell population varies depending on, for instance, individual differences of the pre-amplification-culture or post-amplification-culture cell population and/or the nucleus pulposus tissue from which the cell population is derived. Thus, the ratio depends on the situation, but is, for example, 5% or more, preferably 7% or more, 9% or more, 11% or more, 13% or more, or 15% or more.

In a typical embodiment of the invention, the pre-differentiation-culture cell population is a cell population (post-amplification-culture cell population) obtained through the amplification culture stage in the invention. For example, a cell population containing amplified Tie2-positive stem/progenitor cells is divided at a suitable cell count depending on an embodiment of the differentiation culture stage (e.g., the type and/or size of cultureware) to give a cell population of interest. The cell population obtained through the amplification culture stage in the invention comprises Tie2-positive stem/progenitor cells in the ratio and/or the cell count as described above. In addition, expression of Tie2 in the Tie2-positive stem/progenitor cells is enhanced (Tie2 expression is maintained). Thus, from the viewpoint of enhancing the effects at the differentiation culture stage, the above cell population is preferable as a pre-differentiation-culture cell population.

Note that in some embodiments, the pre-differentiation-culture cell population is not necessarily obtained through the amplification culture stage (the first culture step and/or the second culture step) in the invention. For example, the cell population may be a cell population included in a tissue collected from the body of a human or other animal or a cell population containing target cells obtained by inducing differentiation (via Tie2-positive stem/progenitor cells) of pluripotent or multipotent cells, such as iPS cells or ES cells, which have been produced using cells from a human or other animal.

In the invention, use of the cell population obtained by the third culture method and/or the fourth culture method or use of the cell population obtained by the third culturing step and/or the fourth culturing step at the differentiation culture stage (herein generally referred to as a "post-differentiation-culture cell population) is not particularly limited. The composition of the resulting cell population is adjustable, if appropriate, depending on use thereof. For example, a cell population used for producing a cell preparation for implantation contains as many target cells as possible (e.g., nucleus pulposus cells that produce type II collagen: Col2-positive cells) having functionality useful for exerting a therapeutic or prophylactic effect by implantation. At the same time, it is preferable that the cell population contains some Tie2-positive stem/progenitor cells (e.g., nucleus pulposus stem/progenitor cells) that remain capable of producing such target cells.

The ratio of Col2-positive (nucleus pulposus) cells in the post-differentiation-culture cell population varies depending on, for instance, individual differences of the pre-differentiation-culture cell population and the nucleus pulposus tissue from which the pre-differentiation-culture cell population is derived. Thus, the ratio depends on the situation, but is, for example, 5% or more, preferably 10% or more, 15% or more, 20% or more, 25% or more, or 30% or more.

The ratio of Tie2-positive (nucleus pulposus) stem/progenitor cells in the post-differentiation-culture cell population varies depending on, for instance, individual differences of the pre-differentiation-culture cell population and the nucleus pulposus tissue from which the pre-differentiation-culture cell population is derived. Thus, the ratio depends on the situation, but is, for example, 1% or more, preferably 2% or more, 4% or more, 6% or more, 8% or more, or 10% or more.

Note that in the differentiation culturing step, the number of cells contained in the cell population usually increases. The number of cells (e.g., each of Col2-positive cells or Tie2-positive stem/progenitor cells) in the post-differentiation-culture cell population varies depending on, for instance, individual differences of the pre-differentiation-culture cell population and/or the nucleus pulposus tissue from which the pre-differentiation-culture cell population is derived. Thus, the ratio depends on the situation, but The number of cells is, for example, 2 times or more, 5 times or more, 10 times or more, 20 times or more, 50 times or more, or 100 times or more the number in the pre-differentiation-culture cell population.

### <Culture Medium>

The culture medium used in each culture method or each culturing step in the invention may be any culture medium as long as it is suitable for culturing Tie2-positive stem/progenitor cells and cells differentiated therefrom. A suitable basal culture medium and a suitable additive component(s) may be selected in consideration of the purpose of the culture method or the culturing step. The additive component(s) may be an additive component(s) suitable for amplification culture of Tie2-positive stem/progenitor cells if the culture method is carried out at the time of amplifying Tie2-positive stem/progenitor cells, that is, when the culturing step is at the amplification culture stage. The additive component(s) suitable for inducing differentiation from Tie2-positive stem/progenitor cells into target cells may be selected if the culture method is carried out at the time of inducing differentiation of Tie2-positive stem/progenitor cells, that is, when the culturing step is at the differentiation culture stage.

In the third culture method and the fourth culture method of the invention, or in the third culturing step and the fourth culturing step including the step of performing these methods, it is unnecessary to add, to the culture medium, a component (e.g., methylcellulose) that prevents Tie2-positive stem/progenitor cells and cells differentiated therefrom from attaching to the culture surface of cultureware. That is, the culture medium in the third culture method and the fourth culture method of the invention or in the third culturing step and the fourth culturing step including the step of performing these methods is usually free of any component (e.g., methylcellulose) for preventing cell adhesion to the culture surface of cultureware.

In a representative embodiment of the invention, nucleus pulposus stem/progenitor cells and nucleus pulposus cells differentiated therefrom may be cultured. In this case, the culture medium for each step at the amplification culture stage or the differentiation culture stage may be prepared, for example, by using appropriate amounts of the following basal culture medium, additive component(s), growth factor(s), and other component(s).

Examples of the basal culture medium include DMEM (Dulbecco's Modified Eagle Medium, without or with glucose), αMEM (a-modified Eagle's Minimum Essential Medium), Ham's F-10 medium, Ham's F-12 medium, or a mixture thereof.

Examples of the additive component(s) for amplification culture or differentiation culture include FBS (fetal bovine serum), BSA (bovine serum albumin), L-ascorbic acid (e.g., as L-ascorbic acid magnesium phosphate), selenious acid (e.g., as insulin-transferrin-sodium selenite (ITS: Insulin-Transferrin-Selenium)), and/or 2-mercaptoethanol. If necessary, antibiotics such as penicillin and streptomycin and other component(s) may be further added to the culture medium. Note that the culture medium for amplification culture does not necessarily contain L-ascorbic acid as an additional component.

Examples of the growth factor(s) include FGF (fibroblast growth factor), EGF (epidermal growth factor), and/or Ang-1 (Angiopoietin-1). In an embodiment of the invention, it is preferable to use at least FGF as the growth factor to be added to the culture medium, it is more preferable to use both FGF and EGF, and it is still more preferable to optionally use Ang-1 in addition to FGF and EGF.

Examples of the FGF that can be used include bFGF (basic fibroblast growth factor, sometimes also referred to as FGF-2). The concentration of FGF in the culture medium may be usually in the range of 1 to 50 ng/mL and preferably in the range of 5 to 15 ng/mL, for example, about 10 ng/mL.

Ang-1 is preferably added to a serum-free culture medium. Ang-1 is preferably solubilized in water (soluble Ang-1, recombinant Ang-1). The concentration of Ang-1 (preferably soluble Ang-1) in the culture medium may be usually in the range of 100 to 1000 ng/mL, for example, about 500 ng/mL.

Note that the above growth factors such as FGF, EGF, and Ang-1 are "growth factors having a Tie2 expression-enhancing effect", and can also be interpreted to correspond to a "Tie2 expression enhancer" in a broad sense, but the way of handling these growth factors in the invention is separately described herein.

### <Tie2 Expression Enhancer>

In the second culture method of the invention, at least one kind of "Tie2 expression enhancer" other than growth factors having a Tie2 expression-enhancing effect is added to the culture medium. In particular, when the second culture method is implemented in a step at the amplification culture stage, a Tie2 expression enhancer may be added. This addition exerts an effect of increasing the number of Tie2-positive stem/progenitor cells while the cells remain immature. Further, when a cell population obtained at the amplification culture stage is subjected to the differentiation culturing step, the addition can exert, for instance, an effect of improving a rate of increase in the number of cells in a cell population obtained after the differentiation culturing step and/or a ratio of Tie2-positive stem/progenitor cells and a ratio of functional target cells, and so on. Any one kind of the Tie2 expression enhancer may be used, or two or more kinds thereof may be used in combination. The enhancer may be added to the culture medium in an amount by which the Tie2 activity and effect as described above are elicited.

Examples of the "growth factor(s) having a Tie2 expression-enhancing effect" include Angiopoietin-1 (Ang-1) and/or FGF2 (bFGF). In the second culture method of the invention, at least one kind of "Tie2 expression enhancer" other than growth factors having a Tie2 expression-enhancing effect is added. However, a growth factor(s) having a Tie2 expression-enhancing effect may be optionally used in combination. In particular, the second culture method is implemented in a step at the amplification culture stage. In this case, a growth factor having a Tie2 expression-enhancing effect and another Tie2 expression enhancer, for example, an extract(s) derived from an animal(s) or plant(s) as described below, more preferably an extract(s) derived from a plant(s) may be used in combination. This can exert a synergistic effect. Note that examples of a step at the amplification culture stage include a step that requires use of at least a Tie2 expression enhancer other than growth factors (a growth factor(s) having a Tie2 expression-enhancing effect may be used in combination as an optional component(s)). In addition, it is also possible to perform a step in which only a growth factor(s) having a Tie2 expression-enhancing effect is substantially used as the Tie2 expression enhancer (a step substantially without any Tie2 expression enhancer other than growth factors).

The Tie2 expression enhancer that is other than growth factors and can be used is each animal/plant-derived extract known as a "Tie2 activator" in the art. Examples of such an animal/plant-derived extract(s) include an extract(s) from *Elaeagnus umbellata*, *Lactuca indica, Tamarindus indica L.,* turmeric, yellow wood, *Polygonatum rhizome,* psyllium, *Salsola komarovii,* olive fruit, oysters, camomile, Chinese quince, trichosanthes seed, *Morinda officinalis,* chrysanthemum, *Polygonatum odoratum,* quillaja, ginkgo, *Clerodendrum trichotomum,* Chinese matrimony vine, *Quercus acutissima*, *Alpinia speciosa*, Panax ginseng, *Quercus serrata,* hawthorn, *Pellionia minima, Psidium guajava*, Siberian ginseng, star apple, star fruit, *Gleditsia officinalis Hemsl.,* jujube, cinnamon, wild rocambole, lotus, *Colocasia gigantea*, *Kalopanax pictus,* long pepper, butcher bloom, mango ginger, *Staphylea pinnata*, *Stauntonia hexaphylla, Hemerocallis fulva var. kwanso, Myrica rubra,* Japanese clethra, or rooibos (see Patent Documents 3 to 10). In addition, a component(s) contained in such an extract is, for example, a compound(s) such as ursolic acid, colosolic acid, 3-O-galloylprocyanidin B-1, linolenic acid, 13-hydroxy-9 Z,11E,15E-octadecatrienoic acid, procyanidin B-2, epicatechin-(4β-6)-epicatechin(4β-8)-epicatechin, procyanidin C-1, astragaloside VIII, soya saponin I, 3'-O-methyl gallocatechin, pipernonaline, syringaresinol, 2-methoxycinnamaldehyde, eleutheroside E, eleutheroside E1, sesamin, eudesmin, sylvatesmin, pinoresinol, yangambin, forsythinol, coumarin (see Patent Documents 6 and 12 to 14). They may be used as the Tie2 expression enhancer other than growth factors. For each extract or component, for instance, the usage at which the Tie2 expression-enhancing effect is recognized, the portion (material) of plant/animal and the extraction process suitable for preparation, and/or the procedure for purifying a specific component(s) may also be set based on methods conventionally known to those skilled in the art, if appropriate.

From an industrial point of view, it is advantageous to use, as the Tie2 expression enhancer in the second culturing step in the invention, one or two or more kinds selected from the above animal/plant extracts, more preferably one or two or more kinds selected from the above plant-derived extracts, which are less expensive than growth factors such as Ang-1 and FGF2, preferably have a better Tie2 expression-enhancing effect than those growth factors, and more preferably exhibit a synergistic effect when used in combination with those growth factors.

### - Extract Derived from Plant of Genus Cinnamomum

In a preferred embodiment of the invention, an extract derived from a plant of the genus *Cinnamomum* may be used as the Tie2 expression enhancer. The genus *Cinnamomum* includes 300 or more species such as *Cinnamomumcassia Blume, C. camphora*, *C. daphnoides*, *C. doederleinii, C. japonicum, C. pseudo-pedunculatum, C. sieboldii, C. verum,* or C. *zeylanicum.* For example, an extract of cinnamon branches, which are young branches of cinnamon or a bark of cinnamon, or a product manufactured and sold as cinnamon powder obtained by processing them into powder can be used as an extract derived from a plant of the genus *Cinnamomum* in the invention.

The extract derived from a plant of the genus *Cinnamomum* may be obtained by a conventional procedure, and can be prepared, for example, by immersing or heating, under reflux, a plant body (e.g., cinnamon powder) as a raw material at normal temperature or by heating together with an extraction solvent, and then recovering the supernatant, or by filtering a filtrate and optionally concentrating the filtrate. The extraction solvent used may be a solvent usually used for extraction. Examples include an aqueous solvent such as water, saline, phosphate buffer, or borate buffer. Alternatively, examples include an organic solvent such as an alcohol compound (e.g., ethanol, propylene glycol, 1,3-butylene glycol, glycerin), an aqueous alcohol compound, chloroform, dichloroethane, carbon tetrachloride, acetone, ethyl acetate, or hexane. They may be used singly or may be used in combination. Preferably, water is used as the solvent. The extract obtained by extraction with the above solvent may be used as it is in the form of an extraction liquid. However, from the viewpoint of convenience, the extract may be solidified (pulverized) by, for instance, drying or lyophilization, stored, optionally diluted or re-dissolved (re-dispersed) with a suitable solvent upon use, further optionally subjected to treatment such as filtration, and then used. The extract derived from a plant of the genus *Cinnamomum* may be an extract (purified product) obtained by removing impurities by, for instance, an adsorption process using an ion exchange resin (e.g., a porous polymer such as Amberlite XAD-2), if necessary.

The concentration of the extract derived from a plant of the genus *Cinnamomum* in the culture medium can be adjusted, if appropriate, depending on the properties of the extract to be used, and in consideration of, for instance, the degree of effects as a Tie2 expression enhancer. For example, an extract obtained by extracting 1 mg of cinnamon powder with 1 mL of water (distilled water) may be used as the extract derived from a plant of the genus *Cinnamomum.* In this case, the above extract may be added in an amount of about 1 to 50 v/v%, for example, about 20 v/v% based on the culture medium. Even if the embodiment of extraction and addition is changed, the active ingredient as the Tie2 expression enhancer may be made comparable to that in the embodiment of extraction and addition described above.

### <Extracellular Matrix-Degrading Agent (ECM-Degrading Agent)>

In the fourth culture method of the invention, in order to differentiate Tie2-positive stem/progenitor cells while suppressing the formation of spheroid colonies, an "extracellular matrix-degrading agent (ECM-degrading agent)" is added to the culture medium.

In general, examples of the extracellular matrix (ECM) secreted from stem/progenitor cells or cells differentiated therefrom include collagen, proteoglycan, fibronectin, laminin, tenascin, entactin, elastin, fibrillin, or hyaluronic acid. Collagen includes type I, type II, type III, type IV, type IX (a2), or other types of collagen. Examples of proteoglycan include aggrecan, versican, perlecan (hereinabove, classification is based on the size of the core protein and the number of sugar chains), chondroitin sulfate proteoglycan, heparan sulfate proteoglycan, keratan sulfate proteoglycan, or dermatan sulfate proteoglycan (hereinabove, classification is based on glycosaminoglycan linked to the core protein).

Thus, it is possible to use, as the ECM-degrading agent in the invention, a substance (agent) having activity to degrade ECM as exemplified above and capable of inhibiting the formation of spheroid colonies in accordance with an embodiment of the fourth culture method, that is, in response to the ECM secreted from the cultured Tie2 stem/progenitor cells or cells differentiated therefrom. Any one kind of the ECM-degrading agent may be used, or two or more kinds thereof may be used in combination.

Examples of the typical ECM-degrading agent include proteases having activity to degrade a protein portion(s) constituting the ECM, such as collagenases, which are proteases having activity to degrade collagen. Examples of each collagenase include class I collagenase exhibiting high activity toward high-molecular-weight collagen or class II collagenase exhibiting high activity toward low-molecular-weight collagen fragments. Meanwhile, collagenases from vertebrates cleave collagen in the naturally occurring triple helix region (on a very limited site of α-chain). By contrast, collagenases derived from bacteria act on almost all types of collagen, and can cleave collagen at multiple sites in the triple helix region. The collagenase preparation obtained by concentrating the bacterial culture supernatant contains, in addition to collagenase (e.g., collagenase I, collagenase II), a protease (e.g., neutral protease, clostripain, trypsin, elastase, aminopeptidase) other than collagenase and/or a non-proteolytic enzyme. A preparation, from which a specific component(s) has been removed by, for instance, purification, may also be produced. In the invention, those that are suitable may be selected from, for instance, various known collagenases (preparations) or proteases, and may be used as an ECM-degrading agent.

In a representative embodiment of the fourth culture method of the invention, the Tie2-positive stem/progenitor cells are nucleus pulposus stem/progenitor cells, and the cells (target cells) generated by inducing differentiation from the Tie2-positive stem/progenitor cells are nucleus pulposus cells. The nucleus pulposus cells express, as ECM, type II collagen, for instance, type IX collagen, type XI collagen, and/or proteoglycan. Thus, as the ECM-degrading agent in this embodiment, one having activity to degrade ECM, for example, collagenase having activity to degrade, for instance, type II collagen (or a preparation containing the same) may be selected. Examples of the collagenase (preparation) include "Collagenase P" (derived from *Clostridium histolyticum;* Roche Inc.) or "Liberase" (a mixture of collagenases I and II and neutral protease; Roche Inc.).

Note that the representative ECM-degrading agent may be an enzyme (protein) such as a protease, which has specific activity to degrade proteins contained in ECM, but has low cytotoxicity. Here, it is possible to be able to use, as the ECM-degrading agent, a substance (e.g., a low-molecular-weight compound) other than enzymes (proteins), which substance has a certain level or more of activity to degrade ECM and a certain level or less of cytotoxicity.

The concentration of the ECM-degrading agent in the culture medium may be any concentration that can suppress the formation of spheroid colonies from the cell population containing Tie2 stem/progenitor cells. Depending on the type of ECM-degrading agent used, the fourth culture method may be carried out in a step at the differentiation culture stage (performed as the fourth culturing step). In this case, the concentration may be adjusted, if appropriate, in consideration of the rate of increase in the number of target cells and/or the action on the expression level or positive rate of a predetermined gene(s) (marker gene(s)), and others. For example, if the concentration of the ECM degradation agent is too high, the advantageous effects of the above action may not be sufficiently observed (conversely, may be disadvantageous). Thus, it is preferable to adjust the concentration within a prescribed range depending on the kind of the ECM-degrading agent.

In the method (third-fourth culture method) in which the third culture method and the fourth culture method are fused or the step (third-fourth culturing step) in which the third culturing step and the fourth culturing step are fused, the effects on the rate of increase in the number of target cells, the expression level or positive rate of a predetermined gene(s) (marker gene(s)), or others may vary depending on the combination of the type and concentration of the ECM-degrading agent in the culture medium and the kind of coating agent on the culture surface. Those skilled in the art can set each of the above conditions fit for putting into practice the invention through, for instance, a preliminary test while also considering the properties of the pre-differentiated cell population and other embodiments depending on from what viewpoint the effects are expected.

As described above, the concentration of the ECM-degrading agent in the culture medium is not generally determined, and may be adjusted within the range of, for example, 0.0025 to 5.0 wt%, 0.005 to 2.0 wt%, or 0.01 to 1.0 wt% depending on the combination with the kind of coating agent on the culture surface. In an embodiment of the invention, when "Collagenase P" is used as the ECM-degrading agent, its concentration in the culture medium is adjustable within the range of, for instance, 0.005 to 0.05 wt% or 0.0125 to 0.025 wt%, and is, for example, about 0.0125 wt%. In an embodiment of the invention, when "Liberase" is used as the ECM-degrading agent, its concentration in the culture medium is adjustable within the range of, for instance, 0.25% to 2.0 wt% or 0.5 to 1.0 wt%, and is, for example, about 1.0 wt%.

### <Culture Period and Other Conditions>

Basically, the period and other conditions (e.g., pH, CO₂ level, O₂ level) of each culture method or each culturing step in the invention may be adjusted, if appropriate, so as to obtain a cell population having a desired cell composition (type and number/ratio) according to the purpose of (the culture stage including) the culturing step. The pH may be weakly alkaline (e.g., about 7.15). The CO₂ level may be, for example, about 5%. The O₂ level may be 5% or less (e.g., about 2%). During the period of each culture method or each culturing step (stage), the culture medium may be optionally changed, if appropriate, with a fresh one every predetermined days. Also, the culture medium may be modified or the atmosphere may be changed by adding a component or increasing or decreasing the concentration of the component or the pH after a predetermined number of days has passed.

The period of each of the first culturing step, the second culturing step, or the first-second culturing step in which these steps are fused at the amplification culture stage in the invention is usually about 1 to 3 weeks, for example, about 2 weeks. In addition, the periods of other steps that can be optionally included at the amplification culture stage in the invention are also similar. For example, the period of the culturing step using an FGF-containing culture medium is about one week. When the desired post-amplification-culture cell population is obtained, the amplification culture stage may be terminated. Note that culture (treatment) performed for a short period or a short time (e.g., 24 h or shorter) so that the aim of the amplification culture cannot be achieved fails to correspond to each step performed at the amplification culture stage in the invention.

The period of each of the third culturing step, the fourth culturing step, or the third-fourth culturing step in which the steps are fused at the differentiation culture stage in the invention is usually about 1 to 3 weeks, for example, about 1 to 2 weeks. In addition, the periods of other steps that can be optionally included at the amplification culture stage in the invention are also similar. For example, the period of the culturing step using an FGF-containing culture medium is about one week. Further, the periods of other steps that can be optionally included at the differentiation culture stage in the invention are also similar. When the desired post-differentiation-culture cell population is obtained, the differentiation culture stage may be terminated. Note that culture (treatment) performed for a short period or a short time (e.g., 24 h or shorter) so that the aim of the differentiation culture cannot be achieved fails to correspond to each step performed at the differentiation culture stage in the invention.

### <Cultureware>

Basically, cultureware, a culturing device, and others used in each culture method or each culturing step in the invention may be selected, if appropriate, according to the purpose of (the culture stage including) the culture method or the culturing step so as to obtain a cell population having a desired cell composition (type and number/ratio).

The cultureware used may be cultureware having a common shape, such as a flask, a dish, a plate, or a bag, and may have a well(s) capable of accommodating cells. The cultureware used may be cultureware made of a common material such as glass, plastic, or resin. The surface (culture surface) of the cultureware may be untreated, or may undergo treatment related to cell attachment or other treatment(s). The size (area or volume) of cultureware and, if the cultureware includes wells, the size (aperture and depth) and number of the wells, for instance, may also be selected, if appropriate. If necessary, the cultureware may be shaken or rotated, and the cell population may be cultured while the culture medium is stirred.

In an embodiment of the third culture method (step) and the fourth culture method (step) of the invention, the cultureware and the culturing device may be set according to two-dimensional culture (plate culture). Further, the first culture method (step) of the invention can also be said to be three-dimensional culture from the viewpoint of culturing a cell population while present in a tissue. The cell population-containing tissue (small piece) is placed while suspended in the culture medium. The second culture method (step) of the invention may be an embodiment according to three-dimensional culture when implemented alone. However, the second culture method (step) may be fused to the first culture method (step) so that they are implemented as the first-second culture method (step). In this case, like in the above first culture method (step), the cell population-containing tissue is placed while suspended in the culture medium. In these methods (steps), it is possible to use cultureware having undergone cell attachment-increasing surface treatment as in the third culture method (step). However, there is no problem even if regular cultureware without surface treatment is used.

### <Cell Attachment Treatment>

In the third culture method (step) of the invention, cultureware having undergone cell attachment-increasing surface treatment (herein sometimes referred to as "cell attachment treatment") is used. Typical examples of the cell attachment treatment include treatment in which a coating agent containing an extracellular matrix (ECM) or other biological substance is applied to a culture surface. Examples of the cell attachment treatment also include plasma treatment to modify and make hydrophilic cultureware formed of a low-cell-attachment material, for example, strongly hydrophobic polystyrene.

Examples of the ECM contained in the coating agent for cell attachment treatment include various known ECMs such as collagen (e.g., type I, type II, type IV collagen) or gelatin as a heat-treated product thereof, chondroitin sulfate A, fibronectin, gelatin, laminin, thrombospondin, vitronectin, or proteoglycan (e.g., aggrecan, heparin sulfate proteoglycan). Examples of the biological molecule other than ECM include a polyamino acid such as polylysine (poly-L-lysine or poly-D-lysine). Examples of other coating agents for cell attachment treatment include polyglycolic acid, PLGA (a polylactic acid-glycolic acid copolymer), polyhydroxyalkanoic acid (PHA), poly-ε-caprolactone, polyorthoester, polyacid anhydride, polyphosphazene, polydimethylsiloxane, polyurethane, polytetrafluoroethylene, polyethylene, polysulfone, poly-methyl methacrylate, poly-2 hydroxyethyl methacrylate, polyamide, polypropylene, polyvinyl chloride, polystyrene, polyvinylpyrrolidone, or polyornithine. The coating agent for cell attachment treatment may contain any one of the above-mentioned substances, or may contain two or more kinds thereof.

Here, in the third-fourth culture method (step), as described above, the effects (e.g., the rate of increase in the number of cells in the cell population, ratio of target cells) of the invention may vary depending on the combination of the kind of coating agent for cell attachment treatment and the type and concentration of the ECM-degrading agent added to the culture medium. A cause thereof may be probably because the ECM or other biological substances contained in the coating agent for cell attachment treatment may be affected by the degradation activity by the ECM-degrading agent added to the culture medium. However, an embodiment in which a coating agent for cell attachment treatment and an ECM-degrading agent, which agents may interact in such a manner, are used in combination is also acceptable as long as the effects of the invention are exerted at a certain degree (are not completely blocked). For example, collagenase (preparation) having activity to degrade type II collagen may be added at a predetermined concentration as an ECM-degrading agent to the culture medium. In this case, the coating agent for cell attachment treatment is unlikely to be affected by the type and concentration of the ECM-degrading agent. Alternatively, the coating agent may be used to induce differentiation into target cells (e.g., Col2-positive cells), so that the differentiation can be achieved at a certain level. Preferably, it is preferable to include polylysine (poly-L-lysine or poly-D-lysine) or fibronectin, which are not collagen, or type IV collagen.

The third culture method of the invention can also be carried out at the amplification culture stage. For example, the third culture method may be performed in the step (additional amplification culturing step) of culturing a cell population containing Tie2-positive stem/progenitor cells in a culture medium containing only a growth factor having a Tie2 expression-enhancing effect as a Tie2 expression enhancer described above in relation to the amplification culture stage. Preferable examples of the ECM contained in the coating agent for cell attachment treatment in such an embodiment include gelatin.

### -Composition for Cell Therapy-

The composition for cell therapy according to the invention comprises a cell population obtained by the culture method or the preparation method of the invention as described above, and may optionally comprises another pharmaceutically acceptable component(s).

In a representative embodiment of the invention, the composition for cell therapy is a composition for cell therapy, comprising Col2-positive nucleus pulposus cells differentiated from nucleus pulposus stem/progenitor cells (preferably also comprising Tie2-positive stem/progenitor cells). Examples of an indication for which the composition for cell therapy in this embodiment is indicated, that is, a disease that can be prevented or treated by administering this composition include a disease manifested as a disorder or degeneration of an intervertebral disc (nucleus pulposus) or herniation. Specific examples thereof include discopathy of the lumbar or cervical spine, disc herniation, cervical spondylosis, radiculopathy, spondylolysis/spondylolisthesis, lumbar spinal stenosis, lumbar degenerative spondylolisthesis, or lumbar degenerative scoliosis.

The dosage form of the composition for cell therapy in the invention may be any form as long as the cell population can be transplanted or delivered to a target site (e.g., the nucleus pulposus of an intervertebral disc). Here, the dosage form may be, for example, an injection and preferably an injection for topical administration at or near an intervertebral disc (nucleus pulposus). Alternatively, the dosage form may be an injection for administration into a blood vessel, which makes targeting possible.

Examples of the pharmaceutically acceptable component(s) include water for injection or physiological saline used in the case of preparation as an injection, a culture liquid for the cell population, other suitable solvent/dispersion medium, and/or other additive(s).

The composition for cell therapy according to the invention may be administered in an amount effective in eliciting a desired therapeutic or prophylactic effect. While the ingredient(s) of the composition for cell therapy, the dosage form, the administration subject, the administration route, and other embodiments are considered, such an effective amount may be adjusted, if appropriate, by, for instance, the dose per administration, the number of administrations, and/or the dosing interval (the number of administrations within a certain period). Treatment using the composition for cell therapy according to the invention can be implemented on humans or non-human vertebrates.

### -Preservation Method-

The method of preserving a cell population containing Tie2-positive stem/progenitor cells according to the invention is cryopreservation of the cell population containing Tie2-positive stem/progenitor cells while present in a non-digested tissue. This makes it possible to maintain a state in which Tie2 is activated and/or expressed or prevent a decrease in the number of Tie2-positive stem/progenitor cells in the cell population.

Substantially the same technical matters as those described above in relation to the first culture method is applicable to the technical matters involving the preservation method according to the invention. For example, a cryopreservation procedure and/or an optionally used cryoprotectant may be used for a non-digested tissue containing Tie2-positive stem/progenitor cells. Essentially and substantially the same one is applicable to a conventional cell population containing Tie2-positive stem/progenitor cells isolated from a tissue by digestion treatment.

### [Examples]

A culture medium used for each step at an "amplification culture stage" in the Examples (as referred to as "culture medium for amplification culture stage" in the following Examples) was a culture medium prepared by mixing 60 mL of DMEM (no glucose, Wako) and 40 mL of MEMα (Nacalai Tesque) and by adding 20% of FBS immediately before use while an additional component(s) shown in each table in the Examples was further added (+) or not added (-).

A culture medium used for each step at a "differentiation culture stage" in the Examples (as referred to as "culture medium for differentiation culture stage" in the following Examples) was a culture medium prepared by mixing 60 mL of DMEM (no glucose, Wako) and 40 mL of F10 (Gibco), by adding 1 µL of 2-mercaptoethanol, 6 µL of selenious acid (0.01%), 1.5 mL of ascorbic acid (5 mg/mL), and 5 mL of 30% BSA, and by further adding 30% of FBS immediately before use while an additional component(s) designated in each table in the Examples was added (+) or not added (-).

### [Test Example 1] amplification culture stage: the first culturing step (WTC method)

**[Table 1]**

| Test Example | Amplification culture stage (7 days) | |
|---|---|---|
| | Additional component to prepare a culture medium | Culture method |
| 1-1 | 10ng/mL bFGF | WTC method |
| 1-2 | - | WTC method |
| 1-3 | 10ng/mL bFGF | Two-dimensional culture method |
| 1-4 | - | Two-dimensional culture method |

A nucleus pulposus tissue of an intervertebral disc excised from an affected part of each patient with disc herniation (a 32-year-old woman, a 28-year-old woman, or a 20-year-old man) was finely cut into a size of several-mm cubes using scissors and other instruments. Next, 0.1 to 0.5 g of the finely cut nucleus pulposus tissue containing the cell population was suspended in 3 mL of culture medium prepared such that the additional component designated in Table 1 was added to the culture medium for amplification culture stage. Thereafter, the mixture was dispensed into one well of a 6-well culture dish (the culture surface was untreated), and cultured for 7 days (by WTC method). As a control, the minced nucleus pulposus tissue was not cultured as it was, but digested with collagenase according to a conventional protocol. The resulting isolated cell population was collected. Then, the cell population was cultured while the rest conditions were substantially the same as in the WTC method.

After cultured, the cell population was collected, and the number of cells and the fluorescence intensity of cells positive for Tie2 expression on the cell surface were measured by flow cytometry (FCM). The ratio (Tie2-positive rate) of the number of the cells in the whole cell population and the mean fluorescence intensity (MFI) were then calculated. In the FCM procedure, a fluorescently labeled agent, which was a complex of an anti-human Tie2 antibody and a fluorescent die Allophicocyanin (Anti-Tie-2, Human, Mouse-Mono (87315); Allophicocyanin, Cat#: FAB 3131A; R&D Inc.), was used.

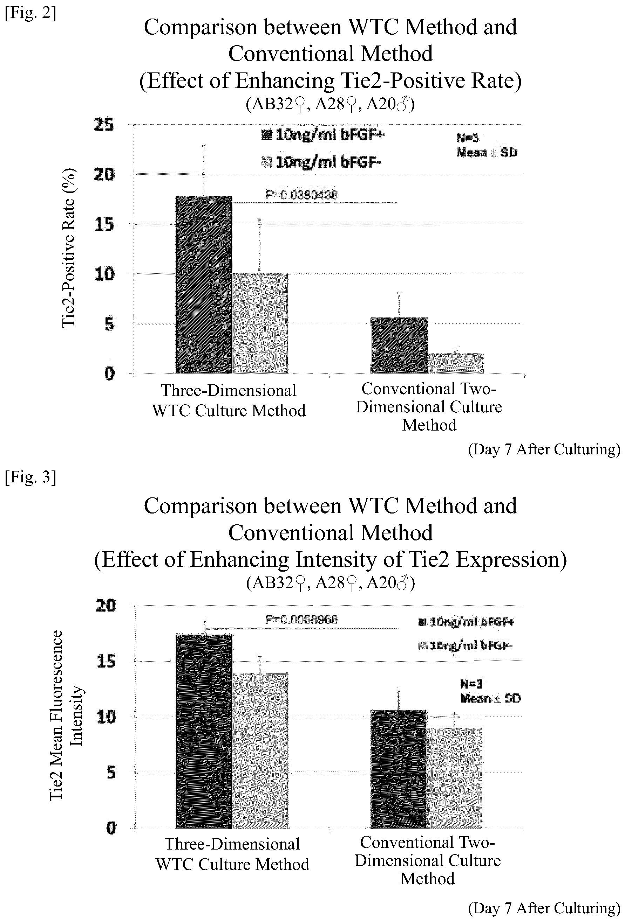

Figs. 2 and 3 show the results. For example, Test Examples 1-1 and 1-3 are compared. Both the results indicate that Test Example 1-1 had significantly higher values (Fig. 2: p < 0.05; Fig. 3: p < 0.01; t-test was used for both).The WTC method was found to exert an effect of enhancing Tie2 expression.

### [Test Example 2] Amplification culture stage (two steps): the first-second culturing step + an additional step

**[Table 2]**

| | | | |
|---|---|---|---|
| | Amplification culture stage | | |

| Test Example | Step 1 (14 days) | | Step 2 (7 days) |
|---|---|---|---|
| | Additional component | Culture method | Additional component |
| 2-1 | 10ng/mL bFGF | WTC method | 10ng/mL bFGF |
| 2-2 | Cinnamon extract | WTC method | 10ng/mL bFGF |

First, 1 mg of commercially available cinnamon powder was suspended in 1 mL of distilled water and extracted overnight at 37°C. The resulting extract (cinnamon extract) was used in this test.

Substantially the same culturing step as in [Test Example 1] (Test Examples 1-1 and 1-2) was repeated except that patients with disc herniation from whom a nucleus pulposus tissue of an intervertebral disc was collected were a 16-year-old woman, a 28-year-old woman, and a 38-year-old woman, a culture medium was prepared such that the additional component designated in Table 2 was added to the culture medium for amplification culture stage, and was used in the first step at the amplification culture stage, and the culture period was 14 days.

After the first culturing step, "Collagenase-P" (final concentration: 0.025%) manufactured by Roche was added to the culture medium to disperse the nucleus pulposus tissue. The cell population separated from the nucleus pulposus tissue was collected and suspended at a density of 1.0 × 10⁴/3 mL in 20% FBS-containing MEM α. Next, the mixture was dispensed into one well of a 6-well culture dish (the culture surface was untreated), and 10 ng/mL bFGF was then added. Subsequently, the cell population was further cultured for 7 days (the total of 21 days).

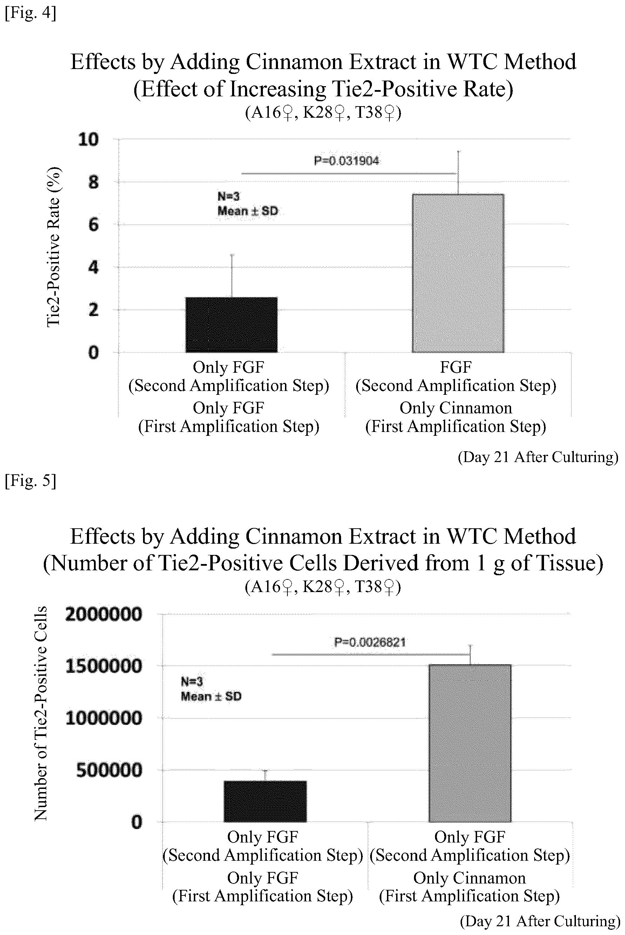

After cultured, the cell population was collected. The FCM procedure like in [Test Example 1] was used to measure each of the rate of cells positive for Tie2 expression on the cell surface or the number of cells derived from 1 g of the tissue. Figs. 4 and 5 show the results.

### [Test Example 3] Amplification culture stage (two steps): the first-second culturing step + an additional step -> differentiation culture stage: the third culturing step

**[Table 3]**

| Test Example | Amplification culture stage | | | Differentiation culture stage (14 days) |
|---|---|---|---|---|
| | Step 1 (14 days) | | Step 2 (7 days) | |
| | Additional component | Culture method | Additional component | Cultureware |
| 3-1 | Cinnamon extract | WTC method | 10ng/mL bFGF | PLL coating |
| 3-2 | Cinnamon extract | WTC method | 10ng/mL bFGF | No coating |

Two steps at the amplification culture stage were performed for a total of 21 days by substantially the same procedure as in Test Example 2 except that disc herniation patients from whom a nucleus pulposus tissue of an intervertebral disc was collected were a 16-year-old women, a 30-year-old man, and a 30-year-old women. After cultured, the cell population was collected. In a step at the differentiation culture stage, a monolayer culture was performed for 14 days on a culture dish coated with poly-L-lysine (PLL) (Test 3-1) or a culture dish without PLL coating (Test Example 3-2).

After cultured, the cell population was collected. Next, the flow cytometry (FCM) was used to measure the number of cells positive for intracellular type II collagen (Col2). Then, the ratio (Col2-positive rate) of the number of the cells in the whole cell population was calculated. The cell population was treated beforehand with a membrane permeation treatment reagent "IntraPrep" (Beckman Coulter, Inc.) so that Col2 in the cells was able to be fluorescently labeled. In the protocol for fluorescently labeling Col2, a mouse anti-human Col2 antibody (Anti-hCL (II) (purified IgG), Cat#: F-57; KYOWA PHARMA CHEMICAL CO., LTD. (old First Fine Chemical, Inc.)) was used as a primary antibody. A complex of a goat anti-mouse IgG antibody and a fluorescent dye FITC (BD, Goat Anti-Mouse Ig FITC, Cat#: 349031) was used as a secondary antibody. Fig. 6 shows the results. In addition, it was assumed that all of 1 g of nucleus pulposus tissue-derived cells were cultured/amplified according to Test Example 2, and then cultured/differentiated according to Test Example 3. The number of Col2-positive cells in this case was calculated. Fig. 7 shows the results. When the third culturing step was applied (Test 3-1), the number of Col2-positive cells was about 3 times higher than in the case where the third culturing step was not applied (Test 3-2).

Note that the FCM procedure was used to measure the number of cells positive for expression of intracellular proteoglycan (PG). Then, the ratio (PG-positive rate) of the number of the cells in the whole cell population was calculated. In the protocol for fluorescently labeling PG, a mouse anti-human PG antibody (Anti-Cartilage Proteoglycan Antibody, adult, clone EFG-4, Cat#: MAB 2015; EMD Millipore) was used as a primary antibody. A complex of a goat anti-mouse IgG antibody and a fluorescent dye FITC (BD, Goat Anti-Mouse Ig FITC, Cat#: 349031) was used as a secondary antibody. As a result, regardless of the application of the third culturing step (PLL coating), the PG positive rate was close to 100% in both cases, and no significant difference was observed (not shown). Unlike the case of proteoglycan, in the case of functional nucleus pulposus cells expressing collagen type II, it was difficult to increase the number of cells in the final cell population by conventional methods. By contrast, this was made possible by a combination of the first-second culturing step and the third culturing step in the invention. This culture method was demonstrated to be superior.

### [Test Example 4] Amplification culture stage (two steps): the first-second culturing step + an additional step -> differentiation culture stage: the third-fourth culturing step

**[Table 4]**

| Test Example | Amplification culture stage | | | Differentiation culture stage (14 days) | |
|---|---|---|---|---|---|
| | Step 1 (14 days) | | Step 2 (7 days) | | |
| | Additional component | Culture method | Additional component | Cultureware | Additional component |
| 4-1 | Cinnamon extract | WTC method | 10ng/mL bFGF | PLL coating | Collagenase- P 0.0125% |
| 4-2 | Cinnamon extract | WTC method | 10ng/mL bFGF | PLL coating | Liberase 0.5% |
| 4-3 | Cinnamon extract | WTC method | 10ng/mL bFGF | PLL coating | None |

Twos steps at the amplification culture stage were performed for a total of 21 days by substantially the same procedure as in Test Example 2. After cultured, the cell population was collected. In a step at the differentiation culture stage, the cell population was cultured for 14 days in a test tube coated with poly-L-lysine (PLL) while a culture medium used was a culture medium for differentiation culture stage, in which medium the additional component designated in Table 4 had been added.

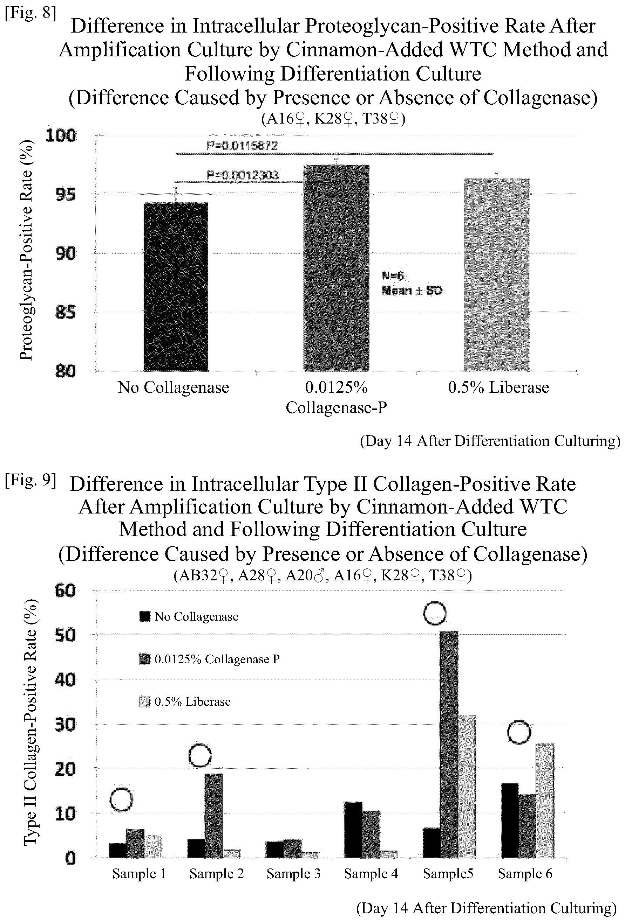

After cultured, the cell population was collected. The PG-positive rate was then calculated in substantially the same manner as in [Test Example 3]. Fig. 8 shows the results. The PG-positive rate was significantly higher in the cases of adding any one of two different collagenases than in the case without adding any collagenase.

For each of Test Examples 4-1 to 4-3, 6 samples were further prepared (patients with disc herniation from whom a nucleus pulposus tissue of an intervertebral disc was collected were a 32-year-old woman, a 28-year-old woman, a 20-year-old man, a 16-year-old woman, a 28-year-old woman, and a 38-year-old woman). The Col2-positive rate was then calculated in substantially the same manner as in [Test Example 3]. Fig. 9 shows the results. There was a difference between samples (difference between individuals from whom a disc nucleus pulposus tissue was collected). The Col2-positive rate was found to be higher in 4 out of 6 samples, that is, in the cases of adding either one or both of the two different collagenases than in the case without adding any collagenase.

### [Test Example 5] Amplification culture stage (two steps): the first-second culturing step + an additional step -> differentiation culture stage: the third-fourth culturing step; part 2

**[Table 5]**

| Test Example | Amplification culture stage | | | Differentiation culture stage (14 days) | |
|---|---|---|---|---|---|
| | Step 1 (14 days) | | Step 2 (7 days) | | |
| | Additional component | Culture method | Additional component | Cultureware | Additional component |
| 5-1 | Cinnamon extract | WTC method | 10ng/mL bFGF | No coating | Collagenase- P 0.025% |
| 5-2 | Cinnamon extract | WTC method | 10ng/mL bFGF | GEL coating | Collagenase- P 0.025% |
| 5-3 | Cinnamon extract | WTC method | 10ng/mL bFGF | Col1 coating | Collagenase- P 0.025% |
| 5-4 | Cinnamon extract | WTC method | 10ng/mL bFGF | Col4 coating | Collagenase- P 0.025% |
| 5-5 | Cinnamon extract | WTC method | 10ng/mL bFGF | FN coating | Collagenase- P 0.025% |
| 5-6 | Cinnamon extract | WTC method | 10ng/mL bFGF | PLL coating | Collagenase- P 0.025% |
| 5-7 | Cinnamon extract | WTC method | 10ng/mL bFGF | No coating | Collagenase- P |
| | | | | | 0.0125% |
| 5-8 | Cinnamon extract | WTC method | 10ng/mL bFGF | GEL coating | Collagenase- P 0.0125% |
| 5-9 | Cinnamon extract | WTC method | 10ng/mL bFGF | Col1 coating | Collagenase- P 0.0125% |
| 5-10 | Cinnamon extract | WTC method | 10ng/mL bFGF | Col4 coating | Collagenase- P 0.0125% |
| 5-11 | Cinnamon extract | WTC method | 10ng/mL bFGF | FN coating | Collagenase- P 0.0125% |
| 5-12 | Cinnamon extract | WTC method | 10ng/mL bFGF | PLL coating | Collagenase- P 0.0125% |

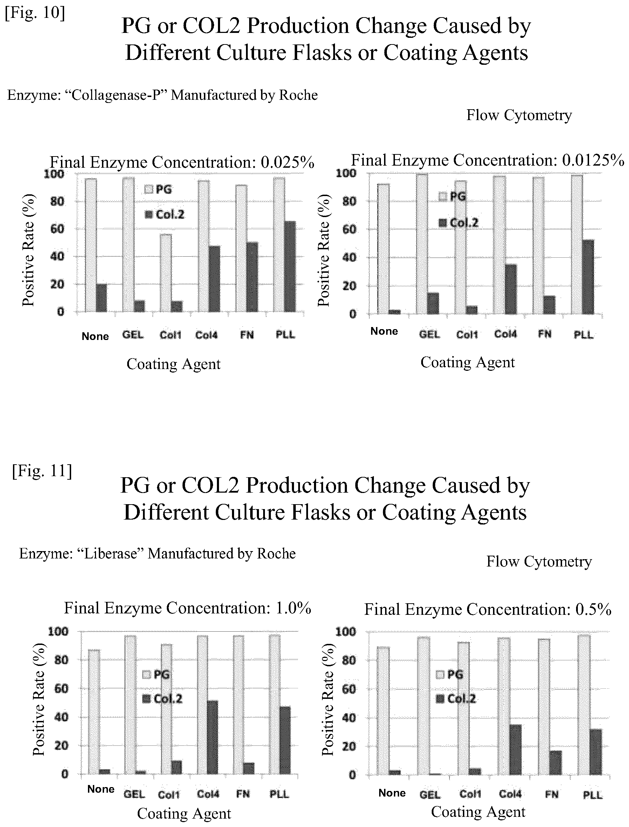

The steps at the amplification culture stage and the differentiation culture stage were performed in substantially the same manner as in Test Example 4 except that the coating agent for cultureware and/or the additional component (Collagenase P) added to the culture medium at the differentiation culture stage were changed as designated in Table 5. Then, the PG-positive rate and the Col2-positive rate were measured. Fig. 10 shows the results. For example, in the case of adding "Collagenase P" to the culture medium, it has been found that use of a coating agent containing Col4 (type IV collagen), FN (fibronectin), or PLL (poly-L-lysine) as a coating agent, particularly a coating agent containing PLL preferably increased the Col2 positive rate although depending on the concentration.

### [Test Example 6] Amplification culture stage (two steps): the first-second culturing step + an additional step -> differentiation culture stage: the third-fourth culturing step; part 3

**[Table 6]**

| Test Example | Amplification culture stage | | | Differentiation culture stage (14 days) | |
|---|---|---|---|---|---|
| | Step 1 (14 days) | | Step 2 (7 days) | | |
| | Additional component | Culture method | Additional component | Cultureware | Additional component |
| 6-1 | Cinnamon extract | WTC method | 10ng/mL bFGF | No coating | Liberase 1.0% |
| 6-2 | Cinnamon extract | WTC method | 10ng/mL bFGF | GEL coating | Liberase 1.0% |
| 6-3 | Cinnamon extract | WTC method | 10ng/mL bFGF | Col1 coating | Liberase 1.0% |
| 6-4 | Cinnamon extract | WTC method | 10ng/mL bFGF | Col4 coating | Liberase 1.0% |
| 6-5 | Cinnamon extract | WTC method | 10ng/mL bFGF | FN coating | Liberase 1.0% |
| 6-6 | Cinnamon extract | WTC method | 10ng/mL bFGF | PLL coating | Liberase 1.0% |
| 6-7 | Cinnamon extract | WTC method | 10ng/mL bFGF | No coating | Liberase 0.5% |
| 6-8 | Cinnamon extract | WTC method | 10ng/mL bFGF | GEL coating | Liberase 0.5% |
| 6-9 | Cinnamon extract | WTC method | 10ng/mL bFGF | Col1 coating | Liberase 0.5% |
| 6-10 | Cinnamon extract | WTC method | 10ng/mL bFGF | Col4 coating | Liberase 0.5% |
| 6-11 | Cinnamon extract | WTC method | 10ng/mL bFGF | FN coating | Liberase 0.5% |
| 6-12 | Cinnamon extract | WTC method | 10ng/mL bFGF | PLL coating | Liberase 0.5% |

The steps at the amplification culture stage and the differentiation culture stage were performed in substantially the same manner as in Test Example 4 except that the coating agent for cultureware and/or the additional component (Liberase) added to the culture medium at the differentiation culture stage were changed as designated in Table 6. Then, the PG-positive rate and the Col2-positive rate were measured. Fig. 11 shows the results. For example, in the case of adding "Liberase" to the culture medium, it has been found that use of a coating agent containing Col4 (type IV collagen) or PLL (poly-L-lysine) as a coating agent preferably increased the Col2 positive rate although depending on the concentration.

### [Test Example 7] Differentiation culture stage: the third culturing step

**[Table 7]**

| | Amplification culture stage | | | |
|---|---|---|---|---|
| Test Example | Step 1 (8-9 days) | | Step 2 (6-8 days) | Differentiation culture stage (6-7 days) |
| | Additional component | Culture method | Additional component | Cultureware |
| 7-1 | 10ng/mL bFGF | Two-dimensional culture method | 10ng/mL bFGF | PLL coating |
| 7-2 | 10ng/mL bFGF | Two-dimensional culture method | 10ng/mL bFGF | No coating |

In this test, similar to the control of Test Example 1 (i.e., the first culture method of the invention: the WTC method was not applied), a cell population isolated from a nucleus pulposus tissue of each patient with disc herniation by digestion treatment using collagenase was used. This cell population was cultured in the 10 ng/mL bFGF-containing culture medium for amplification culture stage (the second culture method of the invention was not applied, and the cinnamon extract as in Test Example 2 was not added) for 8 to 9 days (during the first step) and 6 to 8 days (during the second step).

Subsequently, the cell population containing Ti2-positive stem/progenitor cells that were derived from the nucleus pulposus and amplified and cultured as described above (the first and/or second culture method(s) of the invention was not applied at the amplification culture stage) was subjected to a step based on the third culture method at the differentiation culture stage in the invention. In this step, as in, for instance, Example 3, monolayer culture was performed on a culture dish coated with poly-L-lysine for 6 to 7 days.

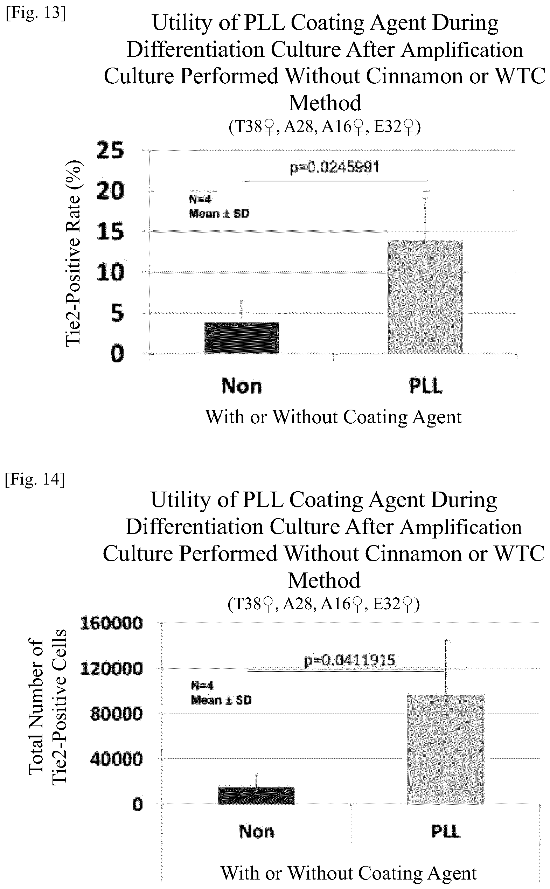

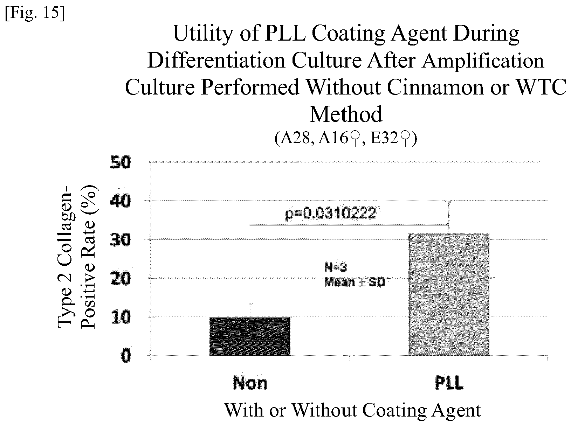

After cultured, the cell population was collected. The Tie2-positive rate and the total number of Tie2-positive cells were measured in substantially the same manner as in Test Examples 1 and 2. In addition, the Col2-positive rate was measured in substantially the same manner as in, for instance, Test Example 3. The results are shown in Fig. 13 (the Tie2-positive rate), Fig. 14 (the total number of Tie2-positive cells), and Fig. 15 (the Col2-positive rate). The third culture method of the invention has been found to exert an effect of increasing the Tie2-positive rate, the total number of Tie2-positive cells, and the Col2-positive rate even in an embodiment in which the third culture method is not used in combination with the first and/or second culture methods.

## Claims

1. A method of culturing a cell population containing stem cells and/or progenitor cells positive for expression of Tie2 (tyrosine kinase with Ig and EGF homology domain-2) (hereinafter referred to as "Tie2-positive stem/progenitor cells"), the method comprising:
culturing the cell population containing Tie2-positive stem/progenitor cells while present in a non-digested tissue (hereinafter, the method is referred to as a "first culture method").

2. The first culture method according to claim 1, wherein the Tie2-positive stem/progenitor cells are Tie2-positive stem/progenitor cells derived from a nucleus pulposus tissue of an intervertebral disc.

3. The first culture method according to claim 1 or 2, wherein the non-digested tissue is a nucleus pulposus tissue of an intervertebral disc.

4. The first culture method according to any one of claims 1 to 3, wherein the non-digested tissue is a tissue obtained by thawing a cryopreserved tissue.

5. The first culture method according to any one of claims 1 to 4, which is performed while the Tie2-positive stem/progenitor cells in the cell population are amplified.

6. A method of culturing a cell population containing Tie2-positive stem/progenitor cells, the method comprising: culturing the cell population containing Tie2-positive stem/progenitor cells in a culture medium containing at least one kind of Tie2 expression enhancer other than growth factors (hereinafter, the method is referred to as a "second culture method").

7. The second culture method according to claim 6, wherein the Tie2 expression enhancer other than growth factors is an animal/plant-derived extract.

8. The second culture method according to claim 7, wherein the plant is a plant of the genus *Cinnamomum.*

9. The second culture method according to any one of claims 6 to 8, which is performed while the Tie2-positive stem/progenitor cells in the cell population are amplified.

10. A method of culturing a cell population containing Tie2-positive stem/progenitor cells, the method comprising:
culturing the cell population containing Tie2-positive stem/progenitor cells by using cultureware with a culture surface having undergone cell attachment-increasing treatment (hereinafter, the method is referred to as a "third culture method").

11. The third culture method according to claim 10, wherein the Tie2-positive stem/progenitor cells have undergone Tie2 expression-enhancing treatment.

12. The third culture method according to claim 10 or 11, wherein the cell attachment-increasing treatment is treatment of applying a coating agent containing an extracellular matrix and/or a polyamino acid.

13. The third culture method according to any one of claims 10 to 12, which is performed while the Tie2-positive stem/progenitor cells in the cell population are differentiated into target cells.

14. The third culture method according to claim 12 or 13, wherein the extracellular matrix and/or the polyamino acid is at least one or more kind selected from the group consisting of type IV collagen, fibronectin, and polylysine.

15. The third culture method according to any one of claims 10 to 14, which is performed while the Tie2-positive stem/progenitor cells in the cell population are amplified.

16. The third culture method according to any one of claims 12 to 15, wherein the extracellular matrix is gelatin.

17. A method of culturing a cell population containing Tie2-positive stem/progenitor cells, the method comprising:
culturing the cell population containing Tie2-positive stem/progenitor cells while suppressing formation of spheroid colonies in a culture medium containing an extracellular matrix-degrading agent (hereinafter, the method is referred to as a "fourth culture method").

18. The fourth culture method according to claim 17, wherein the Tie2-positive stem/progenitor cells have undergone Tie2 expression-enhancing treatment.

19. The fourth culture method according to claim 17 or 18, wherein the extracellular matrix-degrading agent comprises at least a protease having activity to degrade type II collagen.

20. The fourth culture method according to any one of claims 17 to 19, which is performed while the Tie2-positive stem/progenitor cells in the cell population are differentiated into target cells.

21. A method of preparing a cell population containing Tie2-positive stem/progenitor cells, the method comprising:
a culture stage (hereinafter, referred to as an "amplification culture stage") comprising a step of performing the first culture method according to claim 5 and/or the second culture method according to claim 9 to enhance expression of Tie2 in the Tie2-positive stem/progenitor cells and amplify the Tie2-positive stem/progenitor cells in the cell population.

22. The preparation method according to claim 21, wherein the step performed at the amplification culture stage is a step of simultaneously performing the first culture method and the second culture method.

23. The preparation method according to claim 21 or 22, wherein the amplification culture stage further comprises a step of culturing the cell population containing the Tie2-positive stem/progenitor cells in a culture medium only containing, as a Tie2 expression enhancer, a growth factor having a Tie2 expression-enhancing effect.

24. A method for preparing, from a cell population containing Tie2-positive stem/progenitor cells, a cell population containing target cells differentiated from the Tie2-positive stem/progenitor cells, the method comprising:
a culture stage (hereinafter referred to as a "differentiation culture stage") comprising a step of performing the third culture method according to claim 13 or 14 and/or the fourth culture method according to claim 20 to induce differentiation from the Tie2-positive stem/progenitor cells into the target cells.

25. The preparation method according to claim 24, wherein the step performed at the differentiation culture stage is a step of simultaneously performing the third culture method and the fourth culture method.

26. The preparation method according to claim 24 or 25, wherein the target cells are cells expressing at least type II collagen.

27. The preparation method according to claim 26, wherein the cells expressing at least type II collagen are nucleus pulposus cells.

28. The preparation method according to any one of claims 24 to 27, wherein a cell population in which the Tie2-positive stem/progenitor cells remain is obtained through the differentiation culture stage.

29. A method for preparing, from a cell population containing Tie2-positive stem/progenitor cells, a cell population containing target cells differentiated from the Tie2-positive stem/progenitor cells, the method comprising:
the amplification culture stage according to any one of claims 21 to 23; and
the differentiation culture stage according to any one of claims 24 to 28.

30. A cell population obtained by the culture method according to any one of claims 1 to 20.

31. A culture comprising a culture medium in the culture method according to any one of claims 1 to 20 and a cell population to be subjected to the culture method, being cultured, or produced.

32. A cell population obtained through the amplification culture stage and/or the differentiation culture stage in the preparation method according to any one of claims 21 to 29.

33. A culture comprising a culture medium for amplification culture stage or a culture medium for differentiation culture stage and a cell population to be subjected to the amplification culture stage or the differentiation culture stage, respectively, being cultured, or produced in the preparation method according to any one of claims 21 to 29.

34. A composition for cell therapy, comprising the cell population according to claim 30 or 32.

35. The composition for cell therapy according to claim 34 for use in treatment or prevention of a disease having a disorder, degeneration, or herniation of an intervertebral disc as a manifested symptom.

36. A method of preserving a cell population containing Tie2-positive stem/progenitor cells, the method comprising:
cryopreserving the cell population containing Tie2-positive stem/progenitor cells while present in a non-digested tissue to maintain a state in which Tie2 is activated and/or expressed or to suppress a decrease in the Tie2-positive stem/progenitor cells in the cell population.
